# EUROPEAN PATENT APPLICATION

(11) **EP 2 644 619 A1**
(43) Date of publication of application: **02.10.2013**
(21) Application number: 12162642.8
(22) Date of filing: 30.03.2012
(51) Int. Cl.: C07K 14/435

(54) **Monomeric N-terminal spider silk protein domain and uses thereof**

(71) Applicant: Spiber Technologies AB, 756 51 Uppsala (SE)
(72) Inventor: Johansson, Jan, 116 31 Stockholm (SE); Norling, Kerstin, 741 30 KNIVSTA (SE); Rising, Anna, 756 51 Uppsala (SE)
(74) Representative: Henriksson, Mikael

(57) **Abstract**

A novel protein is comprising a moiety of 100-160 amino acid residues having at least 80% identity with the N-terminal moiety of a spider silk protein,
wherein the amino acid residue corresponding to position 72 in SEQ ID NO 1 is not Ala or Gly. The protein moiety is useful in a fusion protein for enhancing the solubility of another moiety in the fusion protein, which is a desired protein or polypeptide. Novel methods are provided for producing such a fusion protein, and optionally a desired protein or polypeptide from said fusion protein.

SEQUENCE LISTING

<110>  SPIBER TECHNOLOGIES AB

<120>  MONOMERIC N-TERMINAL SPIDER SILK PROTEIN DOMAIN AND USES THEREOF

<130>  EP-21055082

<160>  34

<170>  PatentIn version 3.5

<210>  1
<211>  137
<212>  PRT
<213>  Artificial Sequence

<220>
<223>  Derived from Euprosthenops australis

<400>  1

Gly Ser Gly Asn Ser His Thr Thr Pro Trp Thr Asn Pro Gly Leu Ala
1               5                   10                  15

Glu Asn Phe Met Asn Ser Phe Met Gln Gly Leu Ser Ser Met Pro Gly
            20                  25                  30

Phe Thr Ala Ser Gln Leu Asp Asp Met Ser Thr Ile Ala Gln Ser Met
        35                  40                  45

Val Gln Ser Ile Gln Ser Leu Ala Ala Gln Gly Arg Thr Ser Pro Asn
    50                  55                  60

Lys Leu Gln Ala Leu Asn Met Arg Phe Ala Ser Ser Met Ala Glu Ile
65                  70                  75                  80

Ala Ala Ser Glu Glu Gly Gly Gly Ser Leu Ser Thr Lys Thr Ser Ser
                85                  90                  95

Ile Ala Ser Ala Met Ser Asn Ala Phe Leu Gln Thr Thr Gly Val Val
            100                 105                 110

Asn Gln Pro Phe Ile Asn Glu Ile Thr Gln Leu Val Ser Met Phe Ala
        115                 120                 125

Gln Ala Gly Met Asn Asp Val Ser Ala
    130                 135

<210>  2
<211>  411
<212>  DNA
<213>  Artificial Sequence

<220>
<223>  Derived from Euprosthenops australis

<400>  2
ggttctggga attcacacac tacaccatgg acaaacccag gactcgcaga aaacttcatg      60

aacagtttca tgcaaggcct gagctcgatg ccaggtttca cggcaagcca attggatgat     120

atgtcaacca tcgcacaatc catggtacag tcaatacaat ccttggcggc acaaggcagg     180

acatcaccga ataagctgca ggcccttaac atggcgtttg catcttcgat ggcagaaatc     240

gcggcatccg aagaaggagg gggaagcctt tccaccaaaa ctagctctat agccagtgca     300

atgtccaacg cgtttctgca aacaactgga gtggtaaacc aaccgttcat aaatgaaata     360

actcagctcg ttagcatgtt tgctcaagca ggtatgaatg atgtcagtgc t              411

<210>  3
<211>  137
<212>  PRT
<213>  Euprosthenops australis

<400>  3

Gly Ser Gly Asn Ser His Thr Thr Pro Trp Thr Asn Pro Gly Leu Ala
1               5                   10                  15

Glu Asn Phe Met Asn Ser Phe Met Gln Gly Leu Ser Ser Met Pro Gly
            20                  25                  30

Phe Thr Ala Ser Gln Leu Asp Asp Met Ser Thr Ile Ala Gln Ser Met
        35                  40                  45

Val Gln Ser Ile Gln Ser Leu Ala Ala Gln Gly Arg Thr Ser Pro Asn
    50                  55                  60

Lys Leu Gln Ala Leu Asn Met Ala Phe Ala Ser Ser Met Ala Glu Ile
65                  70                  75                  80

Ala Ala Ser Glu Glu Gly Gly Gly Ser Leu Ser Thr Lys Thr Ser Ser
                85                  90                  95

Ile Ala Ser Ala Met Ser Asn Ala Phe Leu Gln Thr Thr Gly Val Val
            100                 105                 110

Asn Gln Pro Phe Ile Asn Glu Ile Thr Gln Leu Val Ser Met Phe Ala
        115                 120                 125

Gln Ala Gly Met Asn Asp Val Ser Ala
    130                 135

<210>  4
<211>  131
<212>  PRT
<213>  Artificial Sequence

<220>
<223>  Consensus NT sequence

<400>  4

Gln Ala Asn Thr Pro Trp Ser Ser Pro Asn Leu Ala Asp Ala Phe Ile
1               5                   10                  15

Asn Ser Phe Met Ser Ala Ala Ser Ser Ser Gly Ala Phe Ser Ala Asp
            20                  25                  30

Gln Leu Asp Asp Met Ser Thr Ile Gly Asp Thr Leu Met Ser Ala Met
        35                  40                  45

Asp Asn Met Gly Arg Ser Gly Lys Ser Thr Lys Ser Lys Leu Gln Ala
    50                  55                  60

Leu Asn Met Ala Phe Ala Ser Ser Met Ala Glu Ile Ala Ala Ala Glu
65                  70                  75                  80

Ser Gly Gly Gly Ser Val Gly Val Lys Thr Asn Ala Ile Ser Asp Ala
                85                  90                  95

Leu Ser Ser Ala Phe Tyr Gln Thr Thr Gly Ser Val Asn Pro Gln Phe
            100                 105                 110

Val Asn Glu Ile Arg Ser Leu Ile Gly Met Phe Ala Gln Ala Ser Ala
        115                 120                 125

Asn Glu Val
    130


<210>  5
<211>  828
<212>  DNA
<213>  Artificial Sequence

<220>
<223>  Fusion protein

<400>  5
atgggccatc atcatcatca tcatatggct agcagcgata aaattattca cctgactgac      60

gacagttttg acacggatgt actcaaagcg gacggggcga tcctcgtcga tttctgggca     120

gagtggtgcg gtccgtgcaa aatgatcgcc ccgattctgg atgaaatcgc tgacgaatat     180

cagggcaaac tgaccgttgc aaaactgaac atcgatcaaa accctggcac tgcgccgaaa     240

tatggcatcc gtggtatccc gactctgctg ctgttcaaaa acggtgaagt ggcggcaacc     300

aaagtgggtg cactgtctaa aggtcagttg aaagagttcc tcgacgctaa cctggccggt     360

tctggttctg gccatatgca ccatcatcat catcattctt ctggtctggt gccacgcggt     420

tctgggaatt cacacactac accatggaca aacccaggac tcgcagaaaa cttcatgaac     480

agtttcatgc aaggcctgag ctcgatgcca ggtttcacgg caagccaatt ggatgatatg 540

tcaaccatcg cacaatccat ggtacagtca atacaatcct tggcggcaca aggcaggaca 600

tcaccgaata agctgcaggc ccttaacatg cgttttgcat cttcgatggc agaaatcgcg 660

gcatccgaag aaggaggggg aagcctttcc accaaaacta gctctatagc cagtgcaatg 720

tccaacgcgt ttctgcaaac aactggagtg gtaaaccaac cgttcataaa tgaaataact 780

cagctcgtta gcatgtttgc tcaagcaggt atgaatgatg tcagtgct 828

<210> 6
<211> 276
<212> PRT
<213> Artificial Sequence

<220>
<223> Fusion protein

<400> 6

Met Gly His His His His His His Met Ala Ser Ser Asp Lys Ile Ile
1               5                   10                  15

His Leu Thr Asp Asp Ser Phe Asp Thr Asp Val Leu Lys Ala Asp Gly
            20                  25                  30

Ala Ile Leu Val Asp Phe Trp Ala Glu Trp Cys Gly Pro Cys Lys Met
        35                  40                  45

Ile Ala Pro Ile Leu Asp Glu Ile Ala Asp Glu Tyr Gln Gly Lys Leu
    50                  55                  60

Thr Val Ala Lys Leu Asn Ile Asp Gln Asn Pro Gly Thr Ala Pro Lys
65                  70                  75                  80

Tyr Gly Ile Arg Gly Ile Pro Thr Leu Leu Leu Phe Lys Asn Gly Glu
                85                  90                  95

Val Ala Ala Thr Lys Val Gly Ala Leu Ser Lys Gly Gln Leu Lys Glu
            100                 105                 110

Phe Leu Asp Ala Asn Leu Ala Gly Ser Gly Ser Gly His Met His His
        115                 120                 125

His His His His Ser Ser Gly Leu Val Pro Arg Gly Ser Gly Asn Ser
    130                 135                 140

His Thr Thr Pro Trp Thr Asn Pro Gly Leu Ala Glu Asn Phe Met Asn
145                 150                 155                 160

Ser Phe Met Gln Gly Leu Ser Ser Met Pro Gly Phe Thr Ala Ser Gln

165                 170                 175

Leu Asp Asp Met Ser Thr Ile Ala Gln Ser Met Val Gln Ser Ile Gln
            180                 185                 190

Ser Leu Ala Ala Gln Gly Arg Thr Ser Pro Asn Lys Leu Gln Ala Leu
        195                 200                 205

Asn Met Arg Phe Ala Ser Ser Met Ala Glu Ile Ala Ala Ser Glu Glu
    210                 215                 220

Gly Gly Gly Ser Leu Ser Thr Lys Thr Ser Ser Ile Ala Ser Ala Met
225                 230                 235                 240

Ser Asn Ala Phe Leu Gln Thr Thr Gly Val Val Asn Gln Pro Phe Ile
                245                 250                 255

Asn Glu Ile Thr Gln Leu Val Ser Met Phe Ala Gln Ala Gly Met Asn
            260                 265                 270

Asp Val Ser Ala
        275

<210>  7
<211>  531
<212>  DNA
<213>  Artificial Sequence

<220>
<223>  Fusion protein

<400>  7
atgggccatc atcatcatca tcatatgagc cataccaccc cgtggaccaa cccgggcctg      60

gcggaaaact ttatgaacag ctttatgcag ggcctgagca gcatgccggg ctttaccgcg     120

agccagctgg atgatatgag caccattgcg cagagcatgg tgcagagcat tcagagcctg     180

gcggcgcagg gccgcaccag cccgaacaaa ctgcaggcgc tgaacatgcg ttttgcgagc     240

agcatggcgg aaattgcggc gagcgaagaa ggcggcggca gcctgagcac caaaaccagc     300

agcattgcga gcgcgatgag caacgcgttt ctgcagacca ccggcgtggt gaaccagccg     360

tttattaacg aaattaccca gctggtgagc atgtttgcgc aggcgggcat gaacgatgtg     420

agcgcgatga ttccgagcag cccggtgcat ctgaaacgcc tgaaactgct gctgctgctg     480

ctgctgctga ttctgctgct gattctgggc gcgctgctgc tgggcctgta a              531

<210>  8
<211>  176
<212>  PRT
<213>  Artificial Sequence

<220>

<223>  Fusion protein

<400>  8

Met Gly His His His His His His Met Ser His Thr Thr Pro Trp Thr
1               5                   10                  15

Asn Pro Gly Leu Ala Glu Asn Phe Met Asn Ser Phe Met Gln Gly Leu
            20                  25                  30

Ser Ser Met Pro Gly Phe Thr Ala Ser Gln Leu Asp Asp Met Ser Thr
        35                  40                  45

Ile Ala Gln Ser Met Val Gln Ser Ile Gln Ser Leu Ala Ala Gln Gly
    50                  55                  60

Arg Thr Ser Pro Asn Lys Leu Gln Ala Leu Asn Met Arg Phe Ala Ser
65                  70                  75                  80

Ser Met Ala Glu Ile Ala Ala Ser Glu Glu Gly Gly Gly Ser Leu Ser
                85                  90                  95

Thr Lys Thr Ser Ser Ile Ala Ser Ala Met Ser Asn Ala Phe Leu Gln
            100                 105                 110

Thr Thr Gly Val Val Asn Gln Pro Phe Ile Asn Glu Ile Thr Gln Leu
        115                 120                 125

Val Ser Met Phe Ala Gln Ala Gly Met Asn Asp Val Ser Ala Met Ile
    130                 135                 140

Pro Ser Ser Pro Val His Leu Lys Arg Leu Lys Leu Leu Leu Leu Leu
145                 150                 155                 160

Leu Leu Leu Ile Leu Leu Leu Ile Leu Gly Ala Leu Leu Leu Gly Leu
                165                 170                 175


<210>  9
<211>  549
<212>  DNA
<213>  Artificial Sequence

<220>
<223>  Fusion protein

<400>  9
atgggccatc atcatcatca tcatatgagc cataccaccc cgtggaccaa cccgggcctg      60

gcggaaaact ttatgaacag ctttatgcag ggcctgagca gcatgccggg ctttaccgcg     120

agccagctgg atgatatgag caccattgcg cagagcatgg tgcagagcat tcagagcctg     180

gcggcgcagg gccgcaccag cccgaacaaa ctgcaggcgc tgaacatgcg ttttgcgagc     240

agcatggcgg aaattgcggc gagcgaagaa ggcggcggca gcctgagcac caaaaccagc 300

agcattgcga gcgcgatgag caacgcgttt ctgcagacca ccggcgtggt gaaccagccg 360

tttattaacg aaattaccca gctggtgagc atgtttgcgc aggcgggcat gaacgatgtg 420

agcgcgggga attcaatgtg ctggctgtgt cgtgcgctga ttaaacgcat ccaggccctg 480

attccgaagg gcggtcgtct gctgccgcaa ctggtgtgcc gtctggttct gcgctgtagc 540

taatgataa 549

<210> 10
<211> 180
<212> PRT
<213> Artificial Sequence

<220>
<223> Fusion protein

<400> 10

Met Gly His His His His His His Met Ser His Thr Thr Pro Trp Thr
1               5                   10                  15

Asn Pro Gly Leu Ala Glu Asn Phe Met Asn Ser Phe Met Gln Gly Leu
            20                  25                  30

Ser Ser Met Pro Gly Phe Thr Ala Ser Gln Leu Asp Asp Met Ser Thr
        35                  40                  45

Ile Ala Gln Ser Met Val Gln Ser Ile Gln Ser Leu Ala Ala Gln Gly
    50                  55                  60

Arg Thr Ser Pro Asn Lys Leu Gln Ala Leu Asn Met Arg Phe Ala Ser
65                  70                  75                  80

Ser Met Ala Glu Ile Ala Ala Ser Glu Glu Gly Gly Gly Ser Leu Ser
                85                  90                  95

Thr Lys Thr Ser Ser Ile Ala Ser Ala Met Ser Asn Ala Phe Leu Gln
            100                 105                 110

Thr Thr Gly Val Val Asn Gln Pro Phe Ile Asn Glu Ile Thr Gln Leu
        115                 120                 125

Val Ser Met Phe Ala Gln Ala Gly Met Asn Asp Val Ser Ala Gly Asn
    130                 135                 140

Ser Met Cys Trp Leu Cys Arg Ala Leu Ile Lys Arg Ile Gln Ala Leu
145                 150                 155                 160

Ile Pro Lys Gly Gly Arg Leu Leu Pro Gln Leu Val Cys Arg Leu Val
                165                 170                 175

Leu Arg Cys Ser
            180

<210>  11
<211>  771
<212>  DNA
<213>  Artificial Sequence

<220>
<223>  Fusion protein

<400>  11
atgggccatc atcatcatca tcatatgagc cataccaccc cgtggaccaa cccgggcctg      60

gcggaaaact ttatgaacag ctttatgcag ggcctgagca gcatgccggg ctttaccgcg     120

agccagctgg atgatatgag caccattgcg cagagcatgg tgcagagcat tcagagcctg     180

gcggcgcagg gccgtaccag cccgaacaaa ctgcaggcgc tgaacatgcg ttttgcgagc     240

agcatggcgg aaattgcggc gagcgaagaa ggcggcggca gcctgagcac caaaaccagc     300

agcattgcga gcgcgatgag caacgcgttt ctgcagacca ccggcgtggt gaaccagccg     360

tttattaacg aaattaccca gctggtgagc atgtttgcgc aggcgggcat gaacgatgtg     420

agcgcgggga attcagagca cctggttacc actgccacct tctccatcgg ctccactggc     480

ctcgtggtgt atgactacca gcagctgctg atcgcctaca agccagcccc tggcacctgc     540

tgctacatca tgaagatagc tccagagagc atccccagtc ttgaggctct cactagaaaa     600

gtccacaact tccagatgga atgctctctg caggccaagc ccgcagtgcc tacgtctaag     660

ctgggccagg cagaggggcg agatgcaggc tcagcaccct ccggagggga cccggccttc     720

ctgggcatgg ccgtgagcac cctgtgtggc gaggtgccgc tctactacat c              771

<210>  12
<211>  257
<212>  PRT
<213>  Artificial Sequence

<220>
<223>  Fusion protein

<400>  12

Met Gly His His His His His His Met Ser His Thr Thr Pro Trp Thr
1               5                   10                  15

Asn Pro Gly Leu Ala Glu Asn Phe Met Asn Ser Phe Met Gln Gly Leu
            20                  25                  30

Ser Ser Met Pro Gly Phe Thr Ala Ser Gln Leu Asp Asp Met Ser Thr
        35                  40                  45

Ile Ala Gln Ser Met Val Gln Ser Ile Gln Ser Leu Ala Ala Gln Gly

50                  55                  60

Arg Thr Ser Pro Asn Lys Leu Gln Ala Leu Asn Met Arg Phe Ala Ser
65                  70                  75                  80

Ser Met Ala Glu Ile Ala Ala Ser Glu Glu Gly Gly Gly Ser Leu Ser
                85                  90                  95

Thr Lys Thr Ser Ser Ile Ala Ser Ala Met Ser Asn Ala Phe Leu Gln
            100                 105                 110

Thr Thr Gly Val Val Asn Gln Pro Phe Ile Asn Glu Ile Thr Gln Leu
        115                 120                 125

Val Ser Met Phe Ala Gln Ala Gly Met Asn Asp Val Ser Ala Gly Asn
    130                 135                 140

Ser Glu His Leu Val Thr Thr Ala Thr Phe Ser Ile Gly Ser Thr Gly
145                 150                 155                 160

Leu Val Val Tyr Asp Tyr Gln Gln Leu Leu Ile Ala Tyr Lys Pro Ala
                165                 170                 175

Pro Gly Thr Cys Cys Tyr Ile Met Lys Ile Ala Pro Glu Ser Ile Pro
            180                 185                 190

Ser Leu Glu Ala Leu Thr Arg Lys Val His Asn Phe Gln Met Glu Cys
        195                 200                 205

Ser Leu Gln Ala Lys Pro Ala Val Pro Thr Ser Lys Leu Gly Gln Ala
    210                 215                 220

Glu Gly Arg Asp Ala Gly Ser Ala Pro Ser Gly Gly Asp Pro Ala Phe
225                 230                 235                 240

Leu Gly Met Ala Val Ser Thr Leu Cys Gly Glu Val Pro Leu Tyr Tyr
                245                 250                 255

Ile

<210>  13
<211>  612
<212>  PRT
<213>  Artificial Sequence

<220>
<223>  Fusion protein

<400>  13

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Ser Glu Pro Lys Leu Ser Leu Ser Phe Ala Leu Ala Asp Gly Ser His
        195                 200                 205

Lys Asn Met Gln Arg Asp Gln Ser Glu Pro Leu Gly Arg Val Leu Ser
    210                 215                 220

Arg Ile Ala Thr Asn Ala Leu Lys Gly His Ala Lys Ala Ala Ala Ala
225                 230                 235                 240

Lys Lys Ser Arg Lys Ser Arg Pro Asn Ala Ser Gly Gly Ala Ala Cys
                245                 250                 255

Ser Gly Pro Gly Pro Glu Pro Ala Val Phe Cys Glu Pro Val Val Lys

260                 265                 270

Leu Tyr Tyr Arg Glu Glu Ala Val Ala Glu Asp Val Leu Asn Val Asp
        275                 280                 285

Ala Trp Gln Asp Gly Ala Val Leu Gln Ile Gly Asp Val Lys Tyr Lys
    290                 295                 300

Val Glu Arg Asn Pro Pro Ala Phe Thr Glu Leu Gln Leu Pro Arg Tyr
305                 310                 315                 320

Ile Met Ala Gly Phe Pro Val Cys Pro Lys Leu Ser Leu Glu Phe Gly
                325                 330                 335

Asp Pro Ala Ser Ser Leu Phe Arg Trp Tyr Lys Glu Ala Lys Pro Gly
            340                 345                 350

Ala Ala Glu Pro Glu Val Gly Val Pro Ser Ser Leu Ser Pro Ser Ser
        355                 360                 365

Pro Ser Ser Ser Trp Thr Glu Thr Asp Val Glu Glu Arg Val Tyr Thr
    370                 375                 380

Pro Ser Asn Ala Asp Ile Gly Leu Arg Leu Lys Leu His Cys Thr Pro
385                 390                 395                 400

Gly Asp Gly Gln Arg Phe Gly His Ser Arg Glu Leu Glu Ser Val Cys
                405                 410                 415

Val Val Glu Ala Gly Pro Gly Thr Cys Thr Phe Asp His Arg His Leu
            420                 425                 430

Tyr Thr Lys Lys Val Thr Glu Asp Ala Leu Ile Arg Thr Val Ser Tyr
        435                 440                 445

Asn Ile Leu Ala Asp Thr Tyr Ala Gln Thr Glu Phe Ser Arg Thr Val
    450                 455                 460

Leu Tyr Pro Tyr Cys Ala Pro Tyr Ala Leu Glu Leu Asp Tyr Arg Gln
465                 470                 475                 480

Asn Leu Ile Gln Lys Glu Leu Thr Gly Tyr Asn Ala Asp Val Ile Cys
                485                 490                 495

Leu Gln Glu Val Asp Arg Ala Val Phe Ser Asp Ser Leu Val Pro Ala
            500                 505                 510

Leu Glu Ala Phe Gly Leu Glu Gly Val Phe Arg Ile Lys Gln His Glu
        515                 520                 525

Gly Leu Ala Thr Phe Tyr Arg Lys Ser Lys Phe Ser Leu Leu Ser Gln
    530                 535                 540

His Asp Ile Ser Phe Tyr Glu Ala Leu Glu Ser Asp Pro Leu His Lys
545                 550                 555                 560

Glu Leu Leu Glu Lys Leu Val Leu Tyr Pro Ser Ala Gln Glu Lys Val
                565                 570                 575

Leu Gln Arg Ser Ser Val Leu Gln Val Ser Val Leu Gln Ser Thr Lys
            580                 585                 590

Asp Ser Ser Lys Arg Ile Cys Val Ala Asn Thr His Leu Tyr Trp His
        595                 600                 605

Pro Lys Gly Arg
    610

<210>  14
<211>  651
<212>  PRT
<213>  Artificial Sequence

<220>
<223>  Fusion protein

<400>  14

<210>  15
<211>  500
<212>  PRT
<213>  Artificial Sequence

<220>
<223>  Fusion protein

<400>  15

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Thr Asp Tyr Asn Pro Asn Tyr Cys Phe Ala Gly Lys Thr Ser Ser Ile
        195                 200                 205

Ser Asp Leu Lys Glu Val Pro Arg Lys Asn Ile Thr Leu Ile Arg Gly
    210                 215                 220

Leu Gly His Gly Ala Phe Gly Glu Val Tyr Glu Gly Gln Val Ser Gly
225                 230                 235                 240

Met Pro Asn Asp Pro Ser Pro Leu Gln Val Ala Val Lys Thr Leu Pro
                245                 250                 255

Glu Val Cys Ser Glu Gln Asp Glu Leu Asp Phe Leu Met Glu Ala Leu
            260                 265                 270

Ile Ile Ser Lys Phe Asn His Gln Asn Ile Val Arg Cys Ile Gly Val
        275                 280                 285

Ser Leu Gln Ser Leu Pro Arg Phe Ile Leu Leu Glu Leu Met Ala Gly
    290                 295                 300

Gly Asp Leu Lys Ser Phe Leu Arg Glu Thr Arg Pro Arg Pro Ser Gln
305                 310                 315                 320

Pro Ser Ser Leu Ala Met Leu Asp Leu Leu His Val Ala Arg Asp Ile
                325                 330                 335

Ala Cys Gly Cys Gln Tyr Leu Glu Glu Asn His Phe Ile His Arg Asp
            340                 345                 350

Ile Ala Ala Arg Asn Cys Leu Leu Thr Cys Pro Gly Pro Gly Arg Val
        355                 360                 365

Ala Lys Ile Gly Asp Phe Gly Met Ala Arg Asp Ile Tyr Arg Ala Ser
    370                 375                 380

Tyr Tyr Arg Lys Gly Gly Cys Ala Met Leu Pro Val Lys Trp Met Pro
385                 390                 395                 400

Pro Glu Ala Phe Met Glu Gly Ile Phe Thr Ser Lys Thr Asp Thr Trp
                405                 410                 415

Ser Phe Gly Val Leu Leu Trp Glu Ile Phe Ser Leu Gly Tyr Met Pro
            420                 425                 430

Tyr Pro Ser Lys Ser Asn Gln Glu Val Leu Glu Phe Val Thr Ser Gly
        435                 440                 445

Gly Arg Met Asp Pro Pro Lys Asn Cys Pro Gly Pro Val Tyr Arg Ile
    450                 455                 460

Met Thr Gln Cys Trp Gln His Gln Pro Glu Asp Arg Pro Asn Phe Ala
465                 470                 475                 480

Ile Ile Leu Glu Arg Ile Glu Tyr Cys Thr Gln Asp Pro Asp Val Ile
                485                 490                 495

Asn Thr Ala Leu
            500

<210>  16
<211>  390
<212>  PRT
<213>  Artificial Sequence

<220>
<223>  Fusion protein

<400>  16

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Glu Ala Ser Pro Ala Ser Gly Pro Arg His Leu Met Asp Pro His Ile
        195                 200                 205

Phe Thr Ser Asn Phe Asn Asn Gly Ile Gly Arg His Lys Thr Tyr Leu
    210                 215                 220

Cys Tyr Glu Val Glu Arg Leu Asp Asn Gly Thr Ser Val Lys Met Asp
225                 230                 235                 240

Gln His Arg Gly Phe Leu His Asn Gln Ala Lys Asn Leu Leu Cys Gly
                245                 250                 255

Phe Tyr Gly Arg His Ala Glu Leu Arg Phe Leu Asp Leu Val Pro Ser
            260                 265                 270

Leu Gln Leu Asp Pro Ala Gln Ile Tyr Arg Val Thr Trp Phe Ile Ser
        275                 280                 285

Trp Ser Pro Cys Phe Ser Trp Gly Cys Ala Gly Glu Val Arg Ala Phe
    290                 295                 300

Leu Gln Glu Asn Thr His Val Arg Leu Arg Ile Phe Ala Ala Arg Ile
305                 310                 315                 320

Tyr Asp Tyr Asp Pro Leu Tyr Lys Glu Ala Leu Gln Met Leu Arg Asp
                325                 330                 335

Ala Gly Ala Gln Val Ser Ile Met Thr Tyr Asp Glu Phe Lys His Cys
            340                 345                 350

Trp Asp Thr Phe Val Asp His Gln Gly Cys Pro Phe Gln Pro Trp Asp
        355                 360                 365

Gly Leu Asp Glu His Ser Gln Ala Leu Ser Gly Arg Leu Arg Ala Ile
    370                 375                 380

Leu Gln Asn Gln Gly Asn
385                 390

<210>  17
<211>  301
<212>  PRT
<213>  Artificial Sequence

<220>
<223>  Fusion protein

<400>  17

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

His Phe Val Asn Ser Trp Val Gln Arg Glu Leu Pro Met Ala Ser Ala
        195                 200                 205

Tyr Cys Asn Asp Ser Ile Phe Ala Tyr Glu Glu Leu Arg Leu Asp Ser
    210                 215                 220

Phe Lys Asp Trp Pro Arg Glu Ser Ala Val Gly Val Ala Ala Leu Ala
225                 230                 235                 240

Lys Ala Gly Leu Phe Tyr Thr Gly Ile Lys Asp Ile Val Gln Cys Phe
                245                 250                 255

Ser Cys Gly Gly Cys Leu Glu Lys Trp Gln Glu Gly Asp Asp Pro Leu
            260                 265                 270

Asp Asp His Thr Arg Cys Phe Pro Asn Cys Pro Phe Leu Gln Asn Met
        275                 280                 285

Lys Ser Ser Ala Glu Val Thr Pro Asp Leu Gln Ser Arg
    290                 295                 300

<210>  18
<211>  684
<212>  PRT
<213>  Artificial Sequence

<220>
<223>  Fusion protein

<400>  18

<210>  19
<211>  584
<212>  PRT
<213>  Artificial Sequence

<220>
<223>  Fusion protein

<400>  19

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Leu Ile Pro Lys Pro Thr Cys Arg Phe Lys Asp Ala Phe Gln Gln Tyr
        195                 200                 205

Pro Asp Leu Leu Lys Ser Ile Ile Arg Val Gly Ile Leu Lys Pro Thr
    210                 215                 220

Pro Ile Gln Ser Gln Ala Trp Pro Ile Ile Leu Gln Gly Ile Asp Leu
225                 230                 235                 240

Ile Val Val Ala Gln Thr Gly Thr Gly Lys Thr Leu Ser Tyr Leu Met
                245                 250                 255

Pro Gly Phe Ile His Leu Asp Ser Gln Pro Ile Ser Arg Glu Gln Arg
            260                 265                 270

Asn Gly Pro Gly Met Leu Val Leu Thr Pro Thr Arg Glu Leu Ala Leu
        275                 280                 285

His Val Glu Ala Glu Cys Ser Lys Tyr Ser Tyr Lys Gly Leu Lys Ser
    290                 295                 300

Ile Cys Ile Tyr Gly Gly Arg Asn Arg Asn Gly Gln Ile Glu Asp Ile
305                 310                 315                 320

Ser Lys Gly Val Asp Ile Ile Ile Ala Thr Pro Gly Arg Leu Asn Asp
                325                 330                 335

Leu Gln Met Asn Asn Ser Val Asn Leu Arg Ser Ile Thr Tyr Leu Val
            340                 345                 350

Ile Asp Glu Ala Asp Lys Met Leu Asp Met Glu Phe Glu Pro Gln Ile
        355                 360                 365

Arg Lys Ile Leu Leu Asp Val Arg Pro Asp Arg Gln Thr Val Met Thr
    370                 375                 380

Ser Ala Thr Trp Pro Asp Thr Val Arg Gln Leu Ala Leu Ser Tyr Leu
385                 390                 395                 400

Lys Asp Pro Met Ile Val Tyr Val Gly Asn Leu Asn Leu Val Ala Val
                405                 410                 415

Asn Thr Val Lys Gln Asn Ile Ile Val Thr Thr Glu Lys Glu Lys Arg
            420                 425                 430

Ala Leu Thr Gln Glu Phe Val Glu Asn Met Ser Pro Asn Asp Lys Val
        435                 440                 445

Ile Met Phe Val Ser Gln Lys His Ile Ala Asp Asp Leu Ser Ser Asp
    450                 455                 460

Phe Asn Ile Gln Gly Ile Ser Ala Glu Ser Leu His Gly Asn Ser Glu
465                 470                 475                 480

Gln Ser Asp Gln Glu Arg Ala Val Glu Asp Phe Lys Ser Gly Asn Ile
                485                 490                 495

Lys Ile Leu Ile Thr Thr Asp Ile Val Ser Arg Gly Leu Asp Leu Asn
            500                 505                 510

Asp Val Thr His Val Tyr Asn Tyr Asp Phe Pro Arg Asn Ile Asp Val
        515                 520                 525

Tyr Val His Arg Val Gly Tyr Ile Gly Arg Thr Gly Lys Thr Gly Thr
    530                 535                 540

Ser Val Thr Leu Ile Thr Gln Arg Asp Ser Lys Met Ala Gly Glu Leu
545                 550                 555                 560

Ile Lys Ile Leu Asp Arg Ala Asn Gln Ser Val Pro Glu Asp Leu Val
                565                 570                 575

Val Met Ala Glu Gln Tyr Lys Leu
            580

<210>  20
<211>  534
<212>  PRT
<213>  Artificial Sequence

<220>
<223>  Fusion protein

<400>  20

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln His His His His His His Ser
                165                 170                 175

Ser Gly Val Asp Leu Gly Thr Leu Glu Ala Leu Phe Gln Gly Gly Ser
            180                 185                 190

Gly Asn Ser His Thr Thr Pro Trp Thr Asn Pro Gly Leu Ala Glu Asn
        195                 200                 205

Phe Met Asn Ser Phe Met Gln Gly Leu Ser Ser Met Pro Gly Phe Thr
    210                 215                 220

Ala Ser Gln Leu Asp Asp Met Ser Thr Ile Ala Gln Ser Met Val Gln
225                 230                 235                 240

Ser Ile Gln Ser Leu Ala Ala Gln Gly Arg Thr Ser Pro Asn Lys Leu
                245                 250                 255

Gln Ala Leu Asn Met Arg Phe Ala Ser Ser Met Ala Glu Ile Ala Ala
            260                 265                 270

Ser Glu Glu Gly Gly Gly Ser Leu Ser Thr Lys Thr Ser Ser Ile Ala
        275                 280                 285

Ser Ala Met Ser Asn Ala Phe Leu Gln Thr Thr Gly Val Val Asn Gln
    290                 295                 300

Pro Phe Ile Asn Glu Ile Thr Gln Leu Val Ser Met Phe Ala Gln Ala
305                 310                 315                 320

Gly Met Asn Asp Val Ser Ala Gly Gly Gly Gly Ser Glu Asn Leu Tyr
                325                 330                 335

Phe Gln Met His His His His His His Ser Ser Gly Val Asp Leu Gly
            340                 345                 350

Thr Glu Asn Leu Tyr Phe Gln Ser Met Gly Asn Leu Asn Leu Val Ala
        355                 360                 365

Val Asn Thr Val Lys Gln Asn Ile Ile Val Thr Thr Glu Lys Glu Lys
    370                 375                 380

Arg Ala Leu Thr Gln Glu Phe Val Glu Asn Met Ser Pro Asn Asp Lys
385                 390                 395                 400

Val Ile Met Phe Val Ser Gln Lys His Ile Ala Asp Asp Leu Ser Ser
                405                 410                 415

Asp Phe Asn Ile Gln Gly Ile Ser Ala Glu Ser Leu His Gly Asn Ser
            420                 425                 430

Glu Gln Ser Asp Gln Glu Arg Ala Val Glu Asp Phe Lys Ser Gly Asn
        435                 440                 445

Ile Lys Ile Leu Ile Thr Thr Asp Ile Val Ser Arg Gly Leu Asp Leu
    450                 455                 460

Asn Asp Val Thr His Val Tyr Asn Tyr Asp Phe Pro Arg Asn Ile Asp
465                 470                 475                 480

Val Tyr Val His Arg Val Gly Tyr Ile Gly Arg Thr Gly Lys Thr Gly
                485                 490                 495

Thr Ser Val Thr Leu Ile Thr Gln Arg Asp Ser Lys Met Ala Gly Glu
            500                 505                 510

Leu Ile Lys Ile Leu Asp Arg Ala Asn Gln Ser Val Pro Glu Asp Leu
        515                 520                 525

Val Val Met Ala Glu Gln
    530

<210>  21
<211>  382
<212>  PRT
<213>  Artificial Sequence

<220>
<223>  Fusion protein

<400>  21

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Gln Ile Val Ser Phe Tyr Arg Val Asn Glu Val Gln Arg Phe Glu Tyr
        195                 200                 205

Ser Arg Pro Ile Arg Lys Gly Glu Lys Asn Pro Asp Asn Glu Phe Ala
    210                 215                 220

Asn Met Trp Ile Glu Arg Thr Ile Tyr Thr Thr Ala Tyr Lys Leu Pro
225                 230                 235                 240

Gly Ile Leu Arg Trp Phe Glu Val Lys Ser Val Phe Met Val Glu Ile
                245                 250                 255

Ser Pro Leu Glu Asn Ala Ile Glu Thr Met Gln Leu Thr Asn Asp Lys
            260                 265                 270

Ile Asn Ser Met Val Gln Gln His Leu Asp Asp Pro Ser Leu Pro Ile
        275                 280                 285

Asn Pro Leu Ser Met Leu Leu Asn Gly Ile Val Asp Pro Ala Val Met
    290                 295                 300

Gly Gly Phe Ala Asn Tyr Glu Lys Ala Phe Phe Thr Asp Arg Tyr Leu
305                 310                 315                 320

Gln Glu His Pro Glu Ala His Glu Lys Ile Glu Lys Leu Lys Asp Leu
                325                 330                 335

Ile Ala Trp Gln Ile Pro Phe Leu Ala Glu Gly Ile Arg Ile His Gly
            340                 345                 350

Asp Lys Val Thr Glu Ala Leu Arg Pro Phe His Glu Arg Met Glu Ala
        355                 360                 365

Cys Phe Lys Gln Leu Lys Glu Lys Val Glu Lys Glu Tyr Gly
    370                 375                 380

<210>  22
<211>  762
<212>  PRT
<213>  Artificial Sequence

<220>
<223>  Fusion protein

<400>  22

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Ser Leu Thr Tyr Lys Glu Ser Gly Val Asp Ile Ala Ala Gly Asn Met
        195                 200                 205

Leu Val Lys Lys Ile Gln Pro Leu Ala Lys Ala Thr Ser Arg Ser Gly
    210                 215                 220

Cys Lys Val Asp Leu Gly Gly Phe Ala Gly Leu Phe Asp Leu Lys Ala
225                 230                 235                 240

Ala Gly Phe Lys Asp Pro Leu Leu Ala Ser Gly Thr Asp Gly Val Gly
                245                 250                 255

Thr Lys Leu Lys Ile Ala Gln Leu Cys Asn Lys His Asp Thr Ile Gly
            260                 265                 270

Gln Asp Leu Val Ala Met Cys Val Asn Asp Ile Leu Ala Gln Gly Ala
        275                 280                 285

Glu Pro Leu Phe Phe Leu Asp Tyr Phe Ser Cys Gly Lys Leu Asp Leu
    290                 295                 300

Ser Val Thr Glu Ala Val Val Ala Gly Ile Ala Lys Ala Cys Gly Lys
305                 310                 315                 320

Ala Gly Cys Ala Leu Leu Gly Gly Glu Thr Ala Glu Met Pro Asp Met
                325                 330                 335

Tyr Pro Pro Gly Glu Tyr Asp Leu Ala Gly Phe Ala Val Gly Ala Met
            340                 345                 350

Glu Arg Asp Gln Lys Leu Pro His Leu Glu Arg Ile Thr Glu Gly Asp
        355                 360                 365

Val Val Val Gly Ile Ala Ser Ser Gly Leu His Ser Asn Gly Phe Ser
    370                 375                 380

Leu Val Arg Lys Ile Val Ala Lys Ser Ser Leu Gln Tyr Ser Ser Pro
385                 390                 395                 400

Ala Pro Asp Gly Cys Gly Asp Gln Thr Leu Gly Asp Leu Leu Leu Thr
                405                 410                 415

Pro Thr Arg Ile Tyr Ser His Ser Leu Leu Pro Val Leu Arg Ser Gly
            420                 425                 430

His Val Lys Ala Phe Ala His Ile Thr Gly Gly Gly Leu Leu Glu Asn
        435                 440                 445

Ile Pro Arg Val Leu Pro Glu Lys Leu Gly Val Asp Leu Asp Ala Gln
    450                 455                 460

Thr Trp Arg Ile Pro Arg Val Phe Ser Trp Leu Gln Gln Glu Gly His
465                 470                 475                 480

Leu Ser Glu Glu Glu Met Ala Arg Thr Phe Asn Cys Gly Val Gly Ala
                485                 490                 495

Val Leu Val Val Ser Lys Glu Gln Thr Glu Gln Ile Leu Arg Gly Ile
            500                 505                 510

Gln Gln His Lys Glu Glu Ala Trp Val Ile Gly Ser Val Val Ala Arg
        515                 520                 525

Ala Glu Gly Ser Pro Arg Val Lys Val Lys Asn Leu Ile Glu Ser Met
    530                 535                 540

Gln Ile Asn Gly Ser Val Leu Lys Asn Gly Ser Leu Thr Asn His Phe
545                 550                 555                 560

Ser Phe Glu Lys Lys Lys Ala Arg Val Ala Val Leu Ile Ser Gly Thr
                565                 570                 575

Gly Ser Asn Leu Gln Ala Leu Ile Asp Ser Thr Arg Glu Pro Asn Ser
            580                 585                 590

Ser Ala Gln Ile Asp Ile Val Ile Ser Asn Lys Ala Ala Val Ala Gly
        595                 600                 605

Leu Asp Lys Ala Glu Arg Ala Gly Ile Pro Thr Arg Val Ile Asn His
    610                 615                 620

Lys Leu Tyr Lys Asn Arg Val Glu Phe Asp Ser Ala Ile Asp Leu Val
625                 630                 635                 640

Leu Glu Glu Phe Ser Ile Asp Ile Val Cys Leu Ala Gly Phe Met Arg
                645                 650                 655

Ile Leu Ser Gly Pro Phe Val Gln Lys Trp Asn Gly Lys Met Leu Asn
            660                 665                 670

Ile His Pro Ser Leu Leu Pro Ser Phe Lys Gly Ser Asn Ala His Glu
        675                 680                 685

Gln Ala Leu Glu Thr Gly Val Thr Val Thr Gly Cys Thr Val His Phe
    690                 695                 700

Val Ala Glu Asp Val Asp Ala Gly Gln Ile Ile Leu Gln Glu Ala Val
705                 710                 715                 720

Pro Val Lys Arg Gly Asp Thr Val Ala Thr Leu Ser Glu Arg Val Lys
                725                 730                 735

Leu Ala Glu His Lys Ile Phe Pro Ala Ala Leu Gln Leu Val Ala Ser
            740                 745                 750

Gly Thr Val Gln Leu Gly Glu Asn Gly Lys
        755                 760

<210> 23
<211> 523
<212> PRT
<213> Artificial Sequence

<220>
<223> Fusion protein

<400> 23

<210>  24
<211>  516
<212>  PRT
<213>  Artificial Sequence

<220>
<223>  Fusion protein

<400>  24

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Asn Glu Asp Leu Gln Val Lys Val Gln Asp Ile Thr Met Gln Trp Tyr
        195                 200                 205

Gln Gln Leu Gln Asp Ala Ser Met Gln Cys Val Leu Thr Phe Glu Gly
    210                 215                 220

Leu Thr Asn Ser Lys Asp Ser Gln Ala Lys Lys Ile Lys Met Asp Leu
225                 230                 235                 240

Gln Lys Ala Ala Thr Ile Pro Val Ser Gln Ile Ser Thr Ile Ala Gly
                245                 250                 255

Ser Lys Leu Lys Glu Ile Phe Asp Lys Ile His Ser Leu Leu Ser Gly
            260                 265                 270

Lys Pro Val Gln Ser Gly Gly Arg Ser Val Ser Val Thr Leu Asn Pro
        275                 280                 285

Gln Gly Leu Asp Phe Val Gln Tyr Lys Leu Ala Glu Lys Phe Val Lys
    290                 295                 300

Gln Gly Glu Glu Glu Val Ala Ser His His Glu Ala Ala Phe Pro Ile
305                 310                 315                 320

Ala Val Val Ala Ser Gly Ile Trp Glu Leu His Pro Arg Val Gly Asp
                325                 330                 335

Leu Ile Leu Ala His Leu His Lys Lys Cys Pro Tyr Ser Val Pro Phe
            340                 345                 350

Tyr Pro Thr Phe Lys Glu Gly Met Ala Leu Glu Asp Tyr Gln Arg Met
        355                 360                 365

Leu Gly Tyr Gln Val Lys Asp Ser Lys Val Glu Gln Gln Asp Asn Phe
    370                 375                 380

Leu Lys Arg Met Ser Gly Met Ile Arg Leu Tyr Ala Ala Ile Ile Gln
385                 390                 395                 400

Leu Arg Trp Pro Tyr Gly Asn Gln Gln Glu Ile His Pro His Gly Leu
                405                 410                 415

Asn His Gly Trp Arg Trp Leu Ala Gln Ile Leu Asn Met Glu Pro Leu
            420                 425                 430

Ser Asp Val Thr Ala Thr Leu Leu Phe Asp Phe Leu Glu Val Cys Gly
        435                 440                 445

Asn Ala Leu Met Lys Gln Tyr Gln Val Gln Phe Trp Lys Met Leu Ile
    450                 455                 460

Leu Ile Lys Glu Asp Tyr Phe Pro Arg Ile Glu Ala Ile Thr Ser Ser
465                 470                 475                 480

Gly Gln Met Gly Ser Phe Ile Arg Leu Lys Gln Phe Leu Glu Lys Cys
                485                 490                 495

Leu Gln His Lys Asp Ile Pro Val Pro Lys Gly Phe Leu Thr Ser Ser
            500                 505                 510

Phe Trp Arg Ser
        515

<210>  25
<211>  866
<212>  PRT
<213>  Artificial Sequence

<220>
<223>  Fusion protein

<400>  25

<210> 26
<211> 346
<212> PRT
<213> Artificial Sequence

<220>
<223> Fusion protein

<400> 26

<210>  27
<211>  466
<212>  PRT
<213>  Artificial Sequence

<220>
<223>  Fusion protein

<400>  27

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Pro Trp Val Gln Leu Ser Gly His Ala Gly Asn Phe Gln Ala Gly Glu
        195                 200                 205

Asp Gly Arg Ile Leu Lys Arg Phe Cys Gln Cys Glu Gln Arg Ser Leu
    210                 215                 220

Glu Gln Leu Met Lys Asp Pro Leu Arg Pro Phe Val Pro Ala Tyr Tyr
225                 230                 235                 240

Gly Met Val Leu Gln Asp Gly Gln Thr Phe Asn Gln Met Glu Asp Leu
                245                 250                 255

Leu Ala Asp Phe Glu Gly Pro Ser Ile Met Asp Cys Lys Met Gly Ser

260                 265                 270

Arg Thr Tyr Leu Glu Glu Glu Leu Val Lys Ala Arg Glu Arg Pro Arg
        275                 280                 285

Pro Arg Lys Asp Met Tyr Glu Lys Met Val Ala Val Asp Pro Gly Ala
    290                 295                 300

Pro Thr Pro Glu Glu His Ala Gln Gly Ala Val Thr Lys Pro Arg Tyr
305                 310                 315                 320

Met Gln Trp Arg Glu Thr Met Ser Ser Thr Ser Thr Leu Gly Phe Arg
                325                 330                 335

Ile Glu Gly Ile Lys Lys Ala Asp Gly Thr Cys Asn Thr Asn Phe Lys
            340                 345                 350

Lys Thr Gln Ala Leu Glu Gln Val Thr Lys Val Leu Glu Asp Phe Val
        355                 360                 365

Asp Gly Asp His Val Ile Leu Gln Lys Tyr Val Ala Cys Leu Glu Glu
    370                 375                 380

Leu Arg Glu Ala Leu Glu Ile Ser Pro Phe Phe Lys Thr His Glu Val
385                 390                 395                 400

Val Gly Ser Ser Leu Leu Phe Val His Asp His Thr Gly Leu Ala Lys
                405                 410                 415

Val Trp Met Ile Asp Phe Gly Lys Thr Val Ala Leu Pro Asp His Gln
            420                 425                 430

Thr Leu Ser His Arg Leu Pro Trp Ala Glu Gly Asn Arg Glu Asp Gly
        435                 440                 445

Tyr Leu Trp Gly Leu Asp Asn Met Ile Cys Leu Leu Gln Gly Leu Ala
    450                 455                 460

Gln Ser
465

<210>  28
<211>  886
<212>  PRT
<213>  Artificial Sequence

<220>
<223>  Fusion protein

<400>  28

<210>  29
<211>  458
<212>  PRT
<213>  Artificial Sequence

<220>
<223>  Fusion protein

<400>  29

<210>  30
<211>  282
<212>  PRT
<213>  Artificial Sequence

<220>
<223>  Fusion protein

<400>  30

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Asp Leu Gln Gly Glu Leu Asp Arg Phe Gly Gly Ile Ser Val Arg Leu
        195                 200                 205

Ala Arg Leu Asp Ala Leu Asp Arg Leu Asp Ala Ala Ala Phe Gln Lys
    210                 215                 220

Gly Leu Gln Ala Ala Val Gln Gln Trp Arg Ser Glu Gly Arg Thr Ala
225                 230                 235                 240

Val Trp Leu His Ile Pro Ile Leu Gln Ser Arg Phe Ile Ala Pro Ala
                245                 250                 255

Ala Ser Leu Gly Phe Cys Phe His His Ala Glu Ser Asp Ser Ser Thr
            260                 265                 270

Leu Thr Leu Trp Leu Arg Glu Gly Pro Ser
        275                 280


<210>  31
<211>  582
<212>  PRT
<213>  Artificial Sequence

<220>
<223>  Fusion protein

<400>  31

<210>  32
<211>  480
<212>  PRT
<213>  Artificial Sequence

<220>
<223>  Fusion protein

<400>  32

His His His His His His Ser Ser Gly Val Asp Leu Gly Thr Leu Glu
1               5                   10                  15

Ala Leu Phe Gln Gly Gly Ser Gly Asn Ser His Thr Thr Pro Trp Thr
            20                  25                  30

Asn Pro Gly Leu Ala Glu Asn Phe Met Asn Ser Phe Met Gln Gly Leu
        35                  40                  45

Ser Ser Met Pro Gly Phe Thr Ala Ser Gln Leu Asp Asp Met Ser Thr
    50                  55                  60

Ile Ala Gln Ser Met Val Gln Ser Ile Gln Ser Leu Ala Ala Gln Gly
65                  70                  75                  80

Arg Thr Ser Pro Asn Lys Leu Gln Ala Leu Asn Met Arg Phe Ala Ser
                85                  90                  95

Ser Met Ala Glu Ile Ala Ala Ser Glu Glu Gly Gly Gly Ser Leu Ser
            100                 105                 110

Thr Lys Thr Ser Ser Ile Ala Ser Ala Met Ser Asn Ala Phe Leu Gln
        115                 120                 125

Thr Thr Gly Val Val Asn Gln Pro Phe Ile Asn Glu Ile Thr Gln Leu
    130                 135                 140

Val Ser Met Phe Ala Gln Ala Gly Met Asn Asp Val Ser Ala Gly Gly
145                 150                 155                 160

Gly Gly Ser Glu Asn Leu Tyr Phe Gln Met His His His His His His
                165                 170                 175

Ser Ser Gly Val Asp Leu Gly Thr Glu Asn Leu Tyr Phe Gln Ser Met
            180                 185                 190

Lys Leu Lys Glu Ile Ser Leu Ser Ala Val Arg Phe Met Glu Glu Leu
        195                 200                 205

Gly Glu Asp Arg Phe Gly Lys Val Tyr Lys Gly His Leu Phe Gly Pro
    210                 215                 220

Ala Pro Gly Glu Gln Thr Gln Ala Val Ala Ile Lys Thr Leu Lys Asp
225                 230                 235                 240

Lys Ala Glu Gly Pro Leu Arg Glu Glu Phe Arg His Glu Ala Met Leu
                245                 250                 255

Arg Ala Arg Leu Gln His Pro Asn Val Val Cys Leu Leu Gly Val Val
            260                 265                 270

Thr Lys Asp Gln Pro Leu Ser Met Ile Phe Ser Tyr Cys Ser His Gly
        275                 280                 285

Asp Leu His Glu Phe Leu Val Met Arg Ser Pro His Ser Asp Val Gly
    290                 295                 300

Ser Thr Asp Asp Asp Arg Thr Val Lys Ser Ala Leu Glu Pro Pro Asp
305                 310                 315                 320

Phe Val His Leu Val Ala Gln Ile Ala Ala Gly Met Glu Tyr Leu Ser
                325                 330                 335

Ser His His Val Val His Lys Asp Leu Ala Thr Arg Asn Val Leu Val
            340                 345                 350

Tyr Asp Lys Leu Asn Val Lys Ile Ser Asp Leu Gly Leu Phe Arg Glu
        355                 360                 365

Val Tyr Ala Ala Asp Tyr Tyr Lys Leu Leu Gly Asn Ser Leu Leu Pro
    370                 375                 380

Ile Arg Trp Met Ala Pro Glu Ala Ile Met Tyr Gly Lys Phe Ser Ile
385                 390                 395                 400

Asp Ser Asp Ile Trp Ser Tyr Gly Val Val Leu Trp Glu Val Phe Ser
                405                 410                 415

Tyr Gly Leu Gln Pro Tyr Cys Gly Tyr Ser Asn Gln Asp Val Val Glu
            420                 425                 430

Met Ile Arg Asn Arg Gln Val Leu Pro Cys Pro Asp Asp Cys Pro Ala
        435                 440                 445

Trp Val Tyr Ala Leu Met Ile Glu Cys Trp Asn Glu Phe Pro Ser Arg
    450                 455                 460

Arg Pro Arg Phe Lys Asp Ile His Ser Arg Leu Arg Ala Trp Gly Asn
465                 470                 475                 480

<210>  33
<211>  615
<212>  PRT
<213>  Artificial Sequence

<220>
<223>  Fusion protein

<400>  33

<210>  34
<211>  670
<212>  PRT
<213>  Artificial Sequence

<220>
<223>  Fusion protein

<400>  34

## Description

### Technical field of the invention

The present invention relates to the field of proteins and polypeptides, and more specifically to expression and production of spider silk proteins (spidroins) and other, non-spidroin proteins and polypeptides. The present invention provides novel proteins which are useful in themselves and as a moiety in novel fusion proteins for expression and production of the desired proteins and polypeptides, as well as nucleic acid molecules encoding these novel proteins and fusion proteins. The present invention also provides a method of expressing and producing a desired protein or polypeptide.

### Background to the invention

Production of proteins and polypeptides from DNA can be achieved in various hosts, but a common problem is the formation of insoluble protein/polypeptide aggregates. This may severely impede or even prevent production of a functional protein/polypeptide. One solution to this problem is to express the desired protein or polypeptide as a fusion protein with a protein or polypeptide that provides the required solubility. The fusion protein may be cleaved, and the desired protein isolated. Alternatively, the desired protein/polypeptide may be maintained integrated in the soluble fusion protein, where it remains functional and can be subjected to further characterization, e.g. activity studies, structure determination and crystallization.

The problem is typically aggravated with low-solubility proteins and polypeptides, e.g. membrane-associated proteins and polypeptides. For instance, lung surfactant protein C (SP C) is a transmembrane protein that is produced by alveolar type II cells and is a constituent of surfactant, that is necessary to prevent alveolar collapse at end expiration. Neonatals often suffer from respiratory distress due to insufficient amounts of surfactant. Today, this condition is treated with surfactant preparations extracted from animal lungs. SP-C33 is a variant of SP-C, where the residues in the transmembrane helix (normally mainly valines) are exchanged for leucines. SP-C33 retains the function of native SP-C, including proper insertion in membranes, but is less prone to aggregate and therefore feasible to produce in large quantities for development of a synthetic surfactant preparation. Since SP-C33 so far has not been possible to produce from DNA, it is today manufactured by chemical synthesis.

Other examples of proteins and polypeptides that pose difficulties when expressed from recombinant DNA are Aβ-peptide, IAPP, PrP, α-synuclein, calcitonin, prolactin, cystatin, ATF and actin; SP-B, α-defensins and β-defensins; class A-H apolipoproteins; LL-37, hCAP18, SP-C, SP-C33Leu, Brichos, GFP, neuroserpin; hormones, including EPO and GH, and growth factors, including IGF-I and IGF-II; avidin and streptavidin; and protease 3C.

WO 2011/115538 discloses a fusion protein comprising a solubility-enhancing moiety which is derived from the N-terminal (NT) fragment of a spider silk protein and a moiety which is a desired protein or polypeptide. A pH above 6.4 is preferred to prevent assembly of the solubility-enhancing moiety.

### Summary of the invention

It is an object of the present invention to provide new means and methods for production of proteins and polypeptides, and in particular for production of proteins and polypeptides with low solubility in water, amyloid-forming proteins and polypeptides, polypeptide drugs and drug targets, disulphide-containing proteins and polypeptides, and apolipoproteins.

It is also an object of the present invention to provide new water-soluble fusion proteins and methods for producing aqueous solutions of such fusions, and in particular fusion proteins comprising proteins and polypeptides with low solubility in water, amyloid-forming proteins and polypeptides, polypeptide drugs and drug targets, disulphide-containing proteins and polypeptides, and apolipoproteins.

It is a specific object of the present invention to provide a novel protein which is derived from the N-terminal (NT) fragment of a spider silk protein and useful e.g. as a solubility-enhancing moiety in a fusion protein.

It is a further object of the present invention to provide a novel protein as set out above which enhances solubility over a wide pH range, including the acidic range.

It is also an object of the present invention to provide a novel protein as set out above which is less prone, or inable, to spontaneously form dimers.

For these and other objects that will be evident from the following specification, the present invention provides according to a first aspect novel proteins as set out in the appended claims.

According to one aspect, the present invention provides compositions comprising aqueous solutions of said proteins as set out in the appended claims.

According to another aspect, the present invention provides isolated nucleic acids encoding the proteins as set out in the appended claims.

According to yet another aspect, the present invention provides useful applications of a novel protein as such and as a moiety in a fusion protein.

According to one aspect, the present invention provides a novel method of producing a desired protein or polypeptide as set out in the appended claims.

According to one related aspect, the present invention provides a novel method of producing a fusion protein comprising a desired protein or polypeptide as set out in the appended claims.

### List of appended sequences

**SEQ ID NO**
   1 NT^{A72R}
   2 NT^{A72R} (DNA)
   3 NT
   4 consensus NT sequence
   5 HisTrxHisNT^{A72R} (DNA)
**SEQ ID NO**
   6 HisTrxHisNT^{A72R}
   7 HisNT^{A72R}MetSP-C33Leu (DNA)
   8 HisNT^{A72R}MetSP-C33Leu
   9 HisNT^{A72R}MetMiniBLeu (DNA)
   10 HisNT^{A72R}MetMiniBLeu
   11 HisNT^{A72R}proSP-C(86-197) (DNA)
   12 HisNT^{A72R}proSP-C(86-197)
   13 NT(A72R)-2PDEA-c202
   14 NT(A72R)-ACOT9A-c009
   15 NT(A72R)-ALKA-c008
   16 NT(A72R)-APOBEC3AA-c002
   17 NT(A72R)-BIRC1A-c050
   18 NT(A72R)-CSADA-c006
   19 NT(A72R)-DDX53A-c006
   20 NT(A72R)-DDX53A-c013
   21 NT(A72R)-DOCK1A-c021
   22 NT(A72R)-GARTA-c015
   23 NT(A72R)-GARTA-c017
   24 NT(A72R)-GLE1A-c007
   25 NT(A72R)-GMPSA-c020
   26 NT(A72R)-ITM2BA-c006
   27 NT(A72R)-ITPKCA-c208
   28 NT(A72R)-MYO10A-c013
   29 NT(A72R)-NUDT6A-c008
   30 NT(A72R)-NUDT6A-c032
   31 NT(A72R)-PIP5K2CA-c002
   32 NT(A72R)-ROR2A-c008
   33 NT(A72R)-SKILA-c017
   34 NT(A72R)-UMPSA-c001

### Brief description of the drawings

Fig. 1 shows a sequence alignment of spidroin N-terminal domains.
Fig. 2 shows the ratio of fluorescence emission at 338 nm and 353 nm vs pH for NT^{A72R} and wild type NT.
Fig. 3 shows deuterium incorporation for each peptic peptide of NT^{A72R} at pH 7.0 and 6.0 as determined by HDX-MS.
Fig. 4 shows a superpositioning of the monomeric and dimeric NT subunit structures.

### Itemized list of embodiments

1. A protein comprising a moiety of 100-160 amino acid residues having at least 80% identity with SEQ ID NO 1, wherein the amino acid residue corresponding to position 72 in SEQ ID NO 1 is not Ala or Gly.
2. A protein according to embodiment 1, wherein the amino acid residue corresponding to position 72 in SEQ ID NO 1 is selected from the group consisting of Arg, Lys, His, Glu, Asp, Gln, Asn, Tyr, Thr and Ser.
3. A protein according to embodiment 2, wherein the amino acid residue corresponding to position 72 in SEQ ID NO 1 is selected from the group consisting of Arg, Lys, His, Glu and Asp.
4. A protein according to embodiment 3, wherein the amino acid residue corresponding to position 72 in SEQ ID NO 1 is Arg.
5. A protein according to any preceding embodiment, wherein the identity with SEQ ID NO 1 is at least 90%.
6. A protein according to any preceding embodiment, wherein the moiety is SEQ ID NO 1.
7. A protein according to any preceding embodiment, which is a fusion protein comprising
   (i) at least one moiety according to any preceding embodiment which is a solubility-enhancing moiety; and
   (ii) at least one moiety which is a desired protein or polypeptide.
8. A fusion protein according to embodiment 7, wherein the fusion protein is comprising at least two moieties according to any one of embodiments 1-6 which are solubility-enhancing moieties.
9. A fusion protein according to embodiment 8, wherein the fusion protein is comprising at least two consecutive moieties according to any one of embodiments 1-6 which are solubility-enhancing moieties.
10. A fusion protein according to any one of embodiments 7-9, wherein at least one solubility-enhancing moiety is linked to the amino-terminal or the carboxy-terminal end of at least one desired protein or polypeptide moiety.
11. A fusion protein according to embodiment 10, wherein at least one solubility-enhancing moiety constitutes the amino-terminal and/or the carboxy-terminal end of the fusion protein.
12. A fusion protein according to any one of embodiments 7-11, further comprising
   (iii) at least one cleavage site arranged between at least one desired protein or polypeptide moiety and at least one solubility-enhancing moiety.
13. A fusion protein according to any one of embodiments 7-12, wherein the desired protein or polypeptide is selected from the group consisting of amyloid-forming proteins and polypeptides, disulphide-containing proteins and polypeptides, apolipoproteins, membrane proteins and polypeptides, protein and polypeptide drugs and drug targets, aggregation-prone proteins and polypeptides, and proteases.
14. A fusion protein according to embodiment 13, wherein the desired protein or polypeptide is selected from the group consisting of Aβ-peptide, IAPP, PrP, α-synuclein, calcitonin, prolactin, cystatin, ATF and actin; SP-B, mini-BLeu, α-defensins and β-defensins; class A-H apolipoproteins; LL-37, hCAP18, SP-C, SP-C33, SP-C33Leu, Brichos, GFP, neuroserpin; hormones, including EPO and GH, and growth factors, including IGF-I and IGF-II; avidin and streptavidin; and protease 3C.
15. A fusion protein according to any one of embodiments 7-14, selected from the group consisting of SEQ ID NOS 6, 8, 10 and 12-34; and proteins having at least 80% identity to any of these proteins.
16. A composition comprising an aqueous solution of a protein according to any one of embodiments 1-15.
17. A composition according to embodiment 16, wherein the pH of said composition is 6.3 or lower, such as 4.2-6.3.
18. An isolated nucleic acid encoding a protein according to any one of embodiments 1-15.
19. An isolated nucleic acid according to embodiment 18, selected from the group consisting of nucleic acids encoding a fusion protein according to embodiment 15 and the group consisting of SEQ ID NOS 2, 5, 7, 9 and 11.
20. Use of at least one moiety according to any one of embodiments 1-6 as a moiety in a fusion protein for enhancing the solubility of another moiety in the fusion protein, which is a desired protein or polypeptide.
21. Use according to embodiment 20, wherein the solubility-enhancing moiety is used for production of the desired protein or polypeptide.
22. Use according to embodiment 20, wherein the solubility-enhancing moiety is used for studying or characterizing the desired protein or polypeptide.
23. Use according to any one of embodiments 20-22, wherein said use involves subjecting the fusion protein to a pH of 6.3 or lower, such as 4.2-6.3.
24. A method of producing a fusion protein, comprising the following steps:
   a) expressing in a suitable host a fusion protein according to any one of embodiments 7-15; and
   b) obtaining a mixture containing the fusion protein, and optionally isolating the fusion protein.
25. A method of producing a desired protein or polypeptide, comprising the following steps:
   a) expressing in a suitable host a fusion protein according to any one of embodiments 12-15;
   b) obtaining a mixture containing the fusion protein or polypeptide, and optionally isolating the fusion protein or polypeptide; and
   c) cleaving the fusion protein to provide the desired protein or polypeptide; and optionally
   d) isolating the desired protein or polypeptide.
26. A method according to any one of embodiments 24-25, wherein at least one step involves subjecting the fusion protein to a pH of 6.3 or lower, such as 4.2-6.3.
27. Method according to any one of embodiments 24-26, wherein step b) further involves purification of the fusion protein on a cation exchange medium.
28. A method according to any one of embodiments 27, wherein the purification of the fusion protein in step b) involves subjecting the fusion protein to a pH of 6.3 or lower, such as 4.2-6.3.
29. A method according to any one of embodiments 27-28, wherein the purification of the fusion protein in step b) occurs in a column, on magnetic beads with functionalized surfaces, or on filters with functionalized surfaces.

### Detailed description of the invention

The present invention is concerned with production and expression of proteins and polypeptides. Depending on the purpose with this production, the end product may vary. It may for instance be desirable to obtain the protein or polypeptide inserted in a lipid membrane, in solution or associated with other biomolecules. It shall also be realized that it may also be highly desirable to obtain the desired protein or polypeptide as part of a fusion protein, which may provide a suitable handle for purification and detection and/or provide desirable properties, e.g. stability and solubility. Maintaining the desired protein or polypeptide functionally integrated in a soluble fusion protein is useful to characterize and study the desired protein or polypeptide.

The present invention is generally based on the insight of the usefulness of a specific variant of the N-terminal (NT) fragment of a spider silk protein due to its capacity to be present as a soluble monomer regardless of the pH of the surrounding aqueous medium. Thus, the present invention provides according to a first aspect a protein comprising a moiety of 100-160 amino acid residues having at least 80% identity with SEQ ID NO 1, wherein the amino acid residue corresponding to position 72 in SEQ ID NO 1 is not Ala or Gly. While this particular amino acid residue is alanine or glycine in known spider silk protein species, it is shown in the Examples that mutation of this particular residue has a critical impact on the pH-dependent dimerization capacity of the NT fragment.

Wildtype NT is highly water-soluble and useful e.g. as a solubility-increasing moiety in a fusion protein for the expression of a desired protein or polypeptide, but it also form dimers at a pH interval of 4.2-6.3 which increases the risk of undesirable aggregation of the fusion proteins. This is a useful pH interval for the functionality and stability of certain desirable proteins and polypeptide. It is also a useful pH interval for certain purification protocols, e.g. when using cation exchange as a purification principle. It has now been realized that replacement of wildtype alanine (or glycine) in the position corresponding to position 72 of wildtype NT with another amino acid, in particular any of the other 18 naturally occurring amino acids, decreases the capacity of the protein to form dimers, without adversely affecting its solubility or its capacity to increase the solubility of a desired protein or polypeptide when they are linked in a fusion protein. The mutant NT protein according to the invention is therefore useful in itself to study the physiologically relevant NT monomer as such. The mutant NT protein according to the invention is also useful as a solubility-increasing moiety in a fusion protein, since it decreases the risk of undesirable aggregation of the fusion proteins, and thereby opens up a new pH window (4.2-6.3) in which derivatives of wildtype NT from spider silk protein can be used in biochemical applications when solubility of protein/polypeptide monomers in aqueous solutions is desirable, e.g. in production or characterization of desirable proteins or polypeptides. Is has surprisingly been determined from experimental data that although this change in position 72 decreases the flexibility of the NT structure and thereby locks the protein into the monomeric state, its capacity to provide stability and solubility to any desirable protein/polypeptide moiety to which it is fused is maintained.

It is preferred that the position corresponding to position 72 of wildtype NT is a charged and/or bulky amino acid selected from the group consisting of Arg, Lys, His, Glu, Asp, Gln, Asn, Tyr, Thr and Ser. In particular, it is preferred that in the position corresponding to position 72 of wildtype NT is a charged amino acid selected from the group consisting of Arg, Lys, His, Glu and Asp, especially the positively charged amino acids Arg, Lys and His. It is particularly preferred that the position corresponding to position 72 of wildtype NT is arginine.

As set out above, the inventive moiety is derived from the NT fragment of a spider silk protein, or spidroin. Although the examples by necessity relate to specific NT fragments, in this case proteins derived from major spidroin 1 (MaSp1) from *Euprosthenops australis,* it is considered that the method disclosed herein is applicable to any similar protein moiety. The terms "spidroins" and "spider silk proteins" are used interchangeably throughout the description and encompass all known spider silk proteins, including major ampullate spider silk proteins which typically are abbreviated "MaSp", or "ADF" in the case of *Araneus diadematus.* These major ampullate spider silk proteins are generally of two types, 1 and 2. These terms furthermore include the new NT protein fragments according to the invention, as defined in the appended claims and itemized embodiments, and other non-natural proteins with a high degree of identity and/or similarity to the known spider silk NT protein fragments.

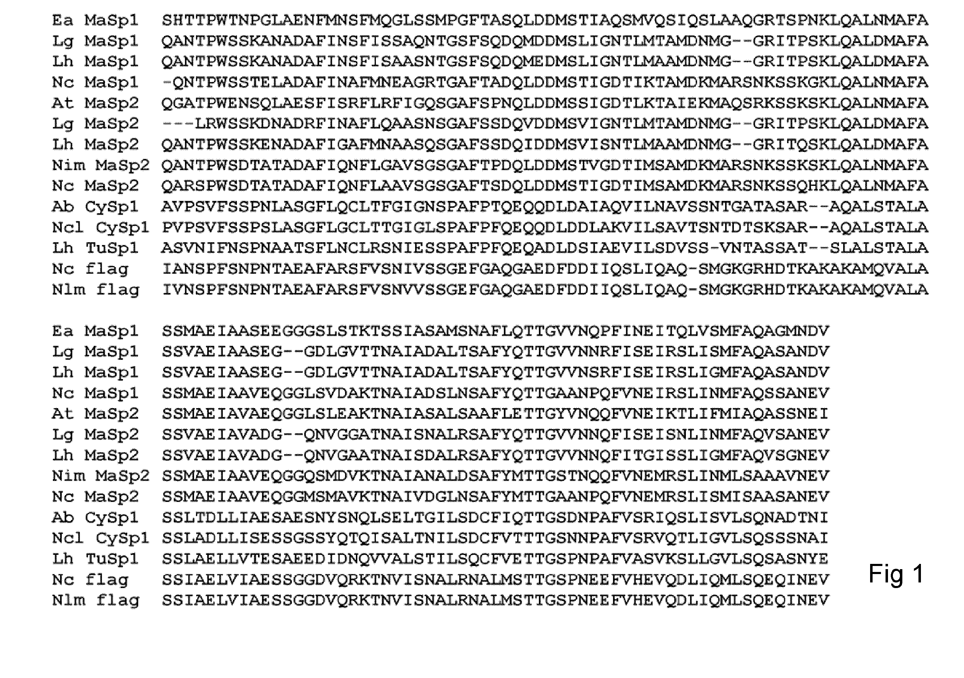

The inventive moiety has a high degree of similarity to the N-terminal (NT) amino acid sequence of spider silk proteins. As shown in Fig 1, this amino acid sequence is well conserved among various species and spider silk proteins, including MaSp1 and MaSp2. The skilled person is therefore well aware how, and to what extent, the amino acid sequence may be varied without departing from the properties and functionality of the N-terminal spider silk protein fragment. In Fig 1, the following spidroin NT fragments are aligned, denoted with GenBank accession entries where applicable:

**TABLE 1 - Spidroin NT fragments**

| **Code** | **Species and spidroin protein** | **GenBank acc. no.** |
|---|---|---|
| Ea MaSp1 | *Euprosthenops australis* MaSp 1 | AM259067 |
| Lg MaSp1 | *Latrodectus geometricus* MaSp 1 | ABY67420 |
| Lh MaSp1 | *Latrodectus hesperus* MaSp 1 | ABY67414 |
| Nc MaSp1 | *Nephila clavipes* MaSp 1 | ACF19411 |
| At MaSp2 | *Argiope trifasciata* MaSp 2 | AAZ15371 |
| Lg MaSp2 | *Latrodectus geometricus* MaSp 2 | ABY67417 |
| Lh MaSp2 | *Latrodectus hesperus* MaSp 2 | ABR68855 |
| Nim MaSp2 | *Nephila inaurata madagascariensis* MaSp 2 | AAZ15322 |
| Nc MaSp2 | *Nephila clavipes* MaSp 2 | ACF19413 |
| Ab CySp1 | *Argiope bruennichi* cylindriform spidroin 1 | BAE86855 |
| Ncl CySp1 | *Nephila clavata* cylindriform spidroin 1 | BAE54451 |
| Lh TuSp1 | *Latrodectus hesperus* tubuliform spidroin | ABD24296 |
| Nc Flag | *Nephila clavipes* flagelliform silk protein | AF027972 |
| Nim Flag | *Nephila inaurata* madagascariensis flagelliform silk protein | AF218623 (translated) |

Only the part corresponding to the N-terminal domain is shown for each sequence, omitting the signal peptide. Nc flag and Nlm flag are translated and edited according to Rising A. et al. Biomacromolecules 7, 3120-3124 (2006)).

It is not critical which specific NT moiety is present in the proteins according to the invention, as long as the NT moiety is not entirely missing. Thus, the NT moiety according to the invention can be selected from any of the amino acid sequences shown in Fig 1 or sequences with a high degree of similarity. A wide variety of sequences can be used in the fusion protein according to the invention. Based on the homologous sequences of Fig 1, the following sequence constitutes a consensus NT amino acid sequence: QANTPWSSPNLADAFINSF(M/L)SA(A/I)SSSGAFSADQLDDMSTIG(D/N/Q)T LMSAMD(N/S/K)MGRSG(K/R)STKSKLQALNMAFASSMAEIAAAESGG(G/Q) SVGVKTNAISDALSSAFYQTTGSVNPQFV(N/S)EIRSLI(G/N)M(F/L)(A/S)QAS ANEV (SEQ ID NO 4).

The sequence of the inventive moiety according to the invention has at least 50% identity, preferably at least 60% identity, to the consensus amino acid sequence SEQ ID NO 4, which is based on the wildtype NT amino acid sequences of Fig 1. In a preferred embodiment, the sequence of the inventive moiety according to the invention has at least 65% identity, preferably at least 70% identity, to the consensus amino acid sequence SEQ ID NO 4. In preferred embodiments, the solubility-enhancing moiety according to the invention has furthermore 70%, preferably 80%, similarity to the consensus amino acid sequence SEQ ID NO 4.

A representative inventive moiety according to the invention is SEQ ID NO 1 (encoded by SEQ ID NO 2), which is derived from the *Euprosthenops australis* NT moiety SEQ ID NO 3 with replacement of alanine in position 72 as set out hereinabove. According to a preferred embodiment of the invention, the inventive moiety has at least 80% identity to SEQ ID NO 1 or any individual amino acid sequence in Fig 1. In preferred embodiments of the invention, the inventive moiety has at least 90%, such as at least 95% identity, to SEQ ID NO 1 or any individual amino acid sequence in Fig 1. In preferred embodiments of the invention, the solubility-enhancing moiety is identical to SEQ ID NO 1 or any individual amino acid sequence in Fig 1, with the proviso that alanine in position 72 is replaced as set out hereinabove.

The term "% identity", as used throughout the specification and the appended claims, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). A comparison is made over the window corresponding to the shortest of the aligned sequences. The amino acid residues at each position are compared, and the percentage of positions in the query sequence that have identical correspondences in the target sequence is reported as % identity.

The term "% similarity", as used throughout the specification and the appended claims, is calculated as described for "% identity", with the exception that the hydrophobic residues Ala, Val, Phe, Pro, Leu, Ile, Trp, Met and Cys are similar; the basic residues Lys, Arg and His are similar; the acidic residues Glu and Asp are similar; and the hydrophilic, uncharged residues Gln, Asn, Ser, Thr and Tyr are similar. The remaining natural amino acid Gly is not similar to any other amino acid in this context.

Throughout this description, alternative embodiments according to the invention fulfill, instead of the specified percentage of identity, the corresponding percentage of similarity. Other alternative embodiments fulfill the specified percentage of identity as well as another, higher percentage of similarity, selected from the group of preferred percentages of identity for each sequence. For example, a sequence may be 70% similar to another sequence; or it may be 70% identical to another sequence; or it may be 70% identical and 90% similar to another sequence.

The inventive moiety contains from 100 to 160 amino acid residues. It is preferred that the inventive moiety contains at least 100, or more than 110, preferably more than 120, amino acid residues. It is also preferred that the inventive moiety contains at most 160, or less than 140 amino acid residues. A typical inventive moiety contains approximately 130-140 amino acid residues.

As set out in detail in WO 2011/115538 which is incorporated in its entirety herein, the N-terminal (NT) fragment of a spider silk protein is particularly useful as a solubility-enhancing moiety in a fusion protein that is produced from recombinant DNA. According to a further aspect, the present invention is further based on the insight of the usefulness of a specific variant of the N-terminal (NT) fragment of a spider silk protein in such a fusion protein due to its capacity to be present as a soluble monomer regardless of the pH of the surrounding aqueous medium.

According to this aspect, the present invention provides a fusion protein comprising (i) at least one solubility-enhancing moiety of 100-160 amino acid residues having at least 80% identity with SEQ ID NO 1, wherein the amino acid residue corresponding to position 72 in SEQ ID NO 1 is not Ala or Gly; and (ii) at least one moiety which is a desired protein or polypeptide. Preferred features of the solubility-enhancing moiety are presented hereinabove. Is has surprisingly been determined from experimental data that although this change in position 72 decreases the flexibility of the NT structure and thereby locks the protein into the monomeric state, its capacity to provide stability and solubility to the desirable protein/polypeptide moiety in the fusion protein according to the invention is maintained.

In a preferred embodiment, the fusion proteins consists of (i) at least one solubility-enhancing moiety of 100-160 amino acid residues having at least 80% identity with SEQ ID NO 1, wherein the amino acid residue corresponding to position 72 in SEQ ID NO 1 is not Ala or Gly; and (ii) at least one moiety which is a desired protein or polypeptide, optionally including other preferred features disclosed herein, e.g. a linker peptide and/or a cleavage site between the solubility-enhancing moiety and the desired protein or polypeptide. In experiments, high yields of different fusion proteins has been achieved in *E. coli.* The fusion protein may be useful as such in isolated form, e.g. for studies of otherwise aggregated or poorly soluble proteins in soluble form, or in crystallization associated with X-ray crystallography. The fusion protein may also be cleaved to release the desired protein or polypeptide.

The term "fusion protein" implies here a protein that is made by expression from a recombinant nucleic acid, i.e. DNA or RNA that is created artificially by combining two or more nucleic acid sequences that would not normally occur together (genetic engineering). The fusion proteins according to the invention are recombinant proteins, and they are therefore not identical to naturally occurring proteins. The combined nucleic acid sequences encode different proteins, partial proteins or polypeptides with certain functional properties. The resulting fusion protein, or recombinant fusion protein, is a single protein with functional properties derived from each of the original proteins, partial proteins or polypeptides.

In certain embodiments, the fusion protein according to the invention and the corresponding genes are chimeric, i.e. the protein/gene fragments are derived from at least two different species. The solubility-enhancing moiety is derived from the N-terminal fragment of a spider silk protein. According to this aspect, it is preferred that, the desired protein or polypeptide is a non-spidroin protein. This implies that the desired protein or polypeptide is not derived from the C-terminal, repetitive or N-terminal fragment of a spider silk protein.

The fusion protein according to the invention may also contain one or more linker peptides. The linker peptide(s) may be arranged between the solubility-enhancing moiety and the desired protein or polypeptide moiety, or may be arranged at either end of the solubility-enhancing moiety and the desired protein or polypeptide moiety. If the fusion protein contains two or more solubility-enhancing moieties, the linker peptide(s) may also be arranged in between two solubility-enhancing moieties. The linker(s) may provide a spacer between the functional units of the fusion protein, but may also constitute a handle for identification and purification of the fusion protein, e.g. a His and/or a Trx tag. If the fusion protein contains two or more linker peptides for identification and purification of the fusion protein, it is preferred that they are separated by a spacer sequence, e.g. His₆-spacer-His₆-. The linker may also constitute a signal peptide, such as a signal recognition particle substrate, which directs the fusion protein to the membrane and/or causes secretion of the fusion protein from the host cell into the surrounding medium. The fusion protein may also include a cleavage site in its amino acid sequence, which allows for cleavage and removal of the linker(s) and/or the solubility-enhancing moiety or moieties. Various cleavage sites are known to the person skilled in the art, e.g. cleavage sites for chemical agents, such as CNBr after Met residues and hydroxylamine between Asn-Gly residues, cleavage sites for proteases, such as thrombin or protease 3C. and self-splicing sequences, such as intein self-splicing sequences.

In one preferred embodiment, the desirable protein/polypeptide is expressed as a C-terminal fusion to His-protease 3C cleavage site-NT^{A72R}-TEV protease cleavage site, see. e.g. Example 5.

Each solubility-enhancing moiety is linked, directly or indirectly, to the desired protein or polypeptide moiety. A direct linkage implies a direct covalent binding between the two moieties without intervening sequences, such as linkers. An indirect linkage also implies that the two moieties are linked by covalent bonds, but that there are intervening sequences, such as linkers and/or one or more further solubility-enhancing moieties.

The at least one solubility-enhancing moiety may be arranged at either end of the desired protein or polypeptide, i.e. C-terminally arranged or N-terminally arranged. It is preferred that the least one solubility-enhancing moiety is arranged at the N-terminal end of the desired protein or polypeptide. If the fusion protein contains one or more linker peptide(s) for identification and purification of the fusion protein, e.g. a His or Trx tag(s), it is preferred that it is arranged at the N-terminal end of the fusion protein. The at least one solubility-enhancing moiety may also be integrated within the desired protein or polypeptide, for instance between domains or parts of a desired protein. In a preferred embodiment, at least one solubility-enhancing moiety constitutes the N-terminal and/or the C-terminal end of the fusion protein, i.e. no linker peptide or other sequence is present terminal of the solubility-enhancing moiety. A typical fusion protein according to the invention may contain 1-6, such as 1-4, such as 1-2 solubility-enhancing moieties.

In a preferred embodiment, the fusion protein is comprising at least two solubility-enhancing moieties, each being derived from the N-terminal (NT) fragment of a spider silk protein as set out hereinabove. The solubility-enhancing moieties, preferably two solubility-enhancing moieties, may be consecutively arranged at either end of the desired protein or polypeptide, i.e. C-terminally arranged or N-terminally arranged. Consecutively arranged solubility-enhancing moieties may also be integrated within the desired protein or polypeptide, for instance between domains or parts of a desired protein. The solubility-enhancing moieties may also be non-consecutively arranged, either at each end of the desired protein or polypeptide, i.e. C-terminally and N-terminally arranged, or at one end of the desired protein or polypeptide and integrated within the desired protein or polypeptide. A typical preferred fusion protein according to the invention may contain 2-6, such as 2-4 solubility-enhancing moieties.

In a preferred embodiment, the fusion protein according to the invention has at least one cleavage site arranged between at least one desired protein or polypeptide moiety and at least one solubility-enhancing moiety. This allows for cleavage of the fusion protein and purification of the desired protein. It is however noted that it may be desirable to obtain the desired protein or polypeptide as part of a fusion protein, which may provide a suitable handle for purification and detection and/or provide desirable properties, e.g. stability and solubility. In this case, the cleavage site may be omitted, or the cleavage site may be included but the cleavage step omitted.

A preferred fusion protein has the form of an N-terminally arranged solubility-enhancing moiety, coupled by a linker peptide of 1-30 amino acid residues, such as 1-10 amino acid residues, to a C-terminally arranged desired protein or polypeptide. The linker peptide may contain a cleavage site. Optionally, the fusion protein has an N-terminal or C-terminal linker peptide, which may contain a purification tag, such as a His tag, and a cleavage site.

Another preferred fusion protein has the form of an N-terminally arranged solubility-enhancing moiety coupled directly to a C-terminally arranged desired protein or polypeptide. Optionally, the fusion protein has an N-terminal or C-terminal linker peptide, which may contain a purification tag, such as a His tag, and a cleavage site.

One preferred fusion protein has the form of a two consecutive N-terminally arranged solubility-enhancing moieties, coupled by a linker peptide of 1-30 amino acid residues, such as 1-10 amino acid residues, to a C-terminally arranged desired protein or polypeptide. The linker peptide may contain a cleavage site. Optionally, the fusion protein has an N-terminal or C-terminal linker peptide, which may contain a purification tag, such as a His tag, and a cleavage site.

Another preferred fusion protein has the form of two consecutive N-terminally arranged solubility-enhancing moieties coupled directly to a C-terminally arranged desired protein or polypeptide. Optionally, the fusion protein has an N-terminal or C-terminal linker peptide, which may contain a purification tag, such as a His tag, and a cleavage site.

In a preferred embodiment, the desired protein or polypeptide is selected from the group consisting of amyloid-forming proteins and polypeptides, disulphide-containing proteins and polypeptides, apolipoproteins, membrane proteins and polypeptides, protein and polypeptide drugs and drug targets, aggregation-prone proteins and polypeptides, and proteases. A preferred group of desired proteins or polypeptides is consisting of Aβ-peptide, IAPP, PrP, α-synuclein, calcitonin, prolactin, cystatin, ATF and actin; SP-B, mini-BLeu, α-defensins and β-defensins; class A-H apolipoproteins; LL-37, hCAP18, SP-C, SP-C33, SP-C33Leu, Brichos, GFP, neuroserpin; hormones, including EPO and GH, and growth factors, including IGF-I and IGF-II; avidin and streptavidin; and protease 3C.

Amyloid-forming proteins and polypeptides according to the invention include proteins and polypeptides that are associated with disease and functional amyloid. Examples of amyloid-forming proteins and polypeptides include amyloid beta peptide (Aβ-peptide), islet amyloid polypeptide (amylin or IAPP), prion protein (PrP), α-synuclein, calcitonin, prolactin, cystatin, atrial natriuretic factor (ATF) and actin. Examples of amyloid-forming proteins and polypeptides according to the invention are listed in Table 2.

**TABLE 2 - Amyloid-forming proteins and Polypeptides**

| **Protein** | **Uniprot ID** |
|---|---|
| Aβ1-42 | P05067 |
| Apolipoprotein SAA | P02735 |
| Cystatin C | P01034 |
| Transthyretin | P02766 |
| Lysozyme | P61626 |
| α-synuclein | P37840 |
| Prion protein | P04156 |
| ODAM | A1E959 |
| Lactadherin | Q08431 |
| Tau | P10636 |
| Gelsolin | P06396 |
| ABri, ADan | Q9Y287 |
| Insulin | P01308 |
| Apolipoprotein A-II | P02652 |
| Apolipoprotein A-IV | P06727 |
| Semenogelin I | P04279 |
| Keratoepithelin | Q15582 |
| Lactotransferrin | P02788 |
| Fibrinogen α-chain | P02671 |
| ANF | P01160 |
| IAPP | P10997 |
| β2-microglobulin | P61769 |
| Calcitonin | P01258 |
| Prolactin | P01236 |
| Apolipoprotein A-I | P02647 |
| CsgA | P28307 |
| Sup35 | C7GN25 |
| Pmel17 | P40967 |
| HET-s | A8HR89 |
| Ure2p | Q8NIE6 |

Examples of disulphide-containing proteins and polypeptides include surfactant protein B (SP-B) and variants thereof, such as Mini-B, Mini-B27, Mini-BLeu, α-defensins and β-defensins. Without being limited to any specific theory, it is contemplated that the solubility-enhancing moiety promotes the desired formation of intrachain disulphide bonds over interchain disulphide bonds in defensins and other disulphide-containing proteins and polypeptides. Examples of disulphide-containing proteins and polypeptides according to the invention are listed in Table 3.

**TABLE 3 - Disulphide-containing proteins and polypeptides**

| **Protein** | **Sequence / Uniprot ID** |
|---|---|
| Human SP-B | FPIPLPYCWLCRALIKRIQAMIPKGALAVAVAQVCRVVPL VAGGICQCLAERYSVILLDTLLGRMLPQLVCRLVLRCSM a |
| Mouse SP-B | LPIPLPFCWLCRTLIKRVQAVIPKGVLAVAVSQVCHVVPL VVGGICQCLAERYTVLLLDALLGRVVPQLVCGLVLRCST a |
| Pig SP-B | FPIPLPFCWLCRTLIKRIQAVVPKGVLLKAVAQVCHVVPL PVGGICQCLAERYIVICLNMLLDRTLPQLVCGLVLRCSS a |
| Rabbit SP-B | FPIPLPLCWLCRTLLKRIQAMIPKGVLAMAVAQVCHVVPL VVGGICQCLAERYTVILLEVLLGHVLPQLVCGLVLRCSS ^a |
| Rat SP-B | LPIPLPFCWLCRTLIKRVQAVIPKGVLAVAVSQVCHVVPL VVGGICQCLAERYTVLLLDALLGRVVPQLVCGLVLRCST a |
| Mini-B | CWLCRALIKRIQAMIPKGGRMLPQLVCRLVLRCS ^{b} |
| Mini-BLeu | CWLCRALIKRIQALIPKGGRLLPQLVCRLVLRCS ^{b} |
| Mini-B27 | CLLCRALIKRFNRYLTPQLVCRLVLRC ^{c} |
| 1 a AA | CWLARALIKRIQALIPKGGRLLPQLVARLVLRCS ^{d} |
| 1b AA | AWLCRALIKRIQALIPKGGRLLPQLVCRLVLRAS ^{e} |
| 1a LL | CWLLRALIKRIQALIPKGGRLLPQLVLRLVLRCS ^{d} |
| 1b LL | LWLCRALIKRIQALIPKGGRLLPQLVCRLVLRLS ^{e} |
| Proinsulin | P01308 |
| CAR D1 ^{f} | P78310 |
| Brichos | SEQ ID NO: 41 |
| ^{a} Cys8-Cys77, Cys11-Cys71, Cys35-Cys46 and intermolecular Cys48-Cys48 linkages ^{b} Cys1-Cys33 and Cys4-Cys27 linkages ^{c} Cys1-Cys27 and Cys4-Cys21 linkages ^{d} Cys1-Cys33 linkage ^{e} Cys4-Cys27 linkage ^{f} Coxsackievirus and adenovirus receptor | |

Examples of apolipoproteins include class A-H apolipoproteins. Examples of apolipoproteins according to the invention are listed in Table 4.

**TABLE 4 - Apolipoproteins**

| **Protein** | **Sequence / Uniprot ID** |
|---|---|
| Apolipoprotein B-100 | P04114 |
| Apolipoprotein C-1 | P02654 |
| Apolipoprotein D | P05090 |
| Apolipoprotein E | P02649 |

Examples of membrane proteins and polypeptides include membrane-associated receptors, including cytokine receptors, KL4, LL-37, hCAP18, surfactant protein C (SP-C) and variants thereof, such as SP-C(Leu), SP-C33, SP-C30 and SP-C33Leu. Other specific examples include SP-C33Leu fused to Mini-B,Mini-BLeu, 1 a AA, 1 b AA, 0 AAAA, 1 a LL, 1 b LL, 0 LLLL or SP-B proteins, optionally via a linker, e.g. Glyₙ, Leuₙ, Gly-Alaₙ or the like. SP-C33Leu may be arranged N-terminal or, preferably, C-terminal to the Mini-B,Mini-BLeu, 1a AA, 1b AA, 0 AAAA, 1a LL, 1 b LL, 0 LLLL or SP-B protein. Examples of membrane proteins and polypeptides according to the invention are listed in Table 5.

**TABLE 5 - Membrane proteins and polypeptides**

| **Protein** | **Sequence** | |
|---|---|---|
| SP-C | FGIPCCPVHLKRLLIVVVVVVLIVVVIVGALLMGL * | |
| SP-C(Leu) | FGIPSSPVHLKRLKLLLLLLLLILLLILGALLMGL | |
| SP-C33 | IPSSPVHLKRLKLLLLLLLLILLLILGALLMGL | |
| SP-C30 | IPSSPVHLKRLKLLLLLLLLILLLILGALL | |
| SP-C33(Leu) | IPSSPVHLKRLKLLLLLLLLILLLILGALLLGL | |
| LL-37 | LLGDFFRKSKEKIGKEFKRIVQRIKDFLRNLVPRTES | |
| KL4 | KLLLLKLLLLKLLLLKLLLLK | |
| * Cys-5 and Cys-6 in native SP-C are palmitoylated | | |
| | | |

| **Protein** | | **Uniprot ID** |
|---|---|---|
| Growth hormone receptor | | P10912 |
| G-protein coupled receptor 35 | | Q9HC97 |
| Insulin receptor, | | P06213 |
| Gonadotropin releasing hormone receptor | | P30968 |
| Very low density lipoprotein receptor | | P98155 |
| TGF-beta receptor, type 1 | | P36897 |
| Prostaglandin D2 receptor | | Q13258 |
| Receptor tyrosine-protein kinase erbB-2 (HER2) | | P04626 |
| Receptor tyrosine-protein kinase erbB-4 (HER4) | | Q15303 |
| Receptor tyrosine-protein kinase erbB-3 (HER3) | | P21860 |
| Aquaporin-1 | | P29972 |
| Aquaporin-2 | | P41181 |
| Chloride channel protein CIC-Ka | | P51800 |
| Chloride channel protein CIC-Kb | | P51801 |
| Integral membrane protein DGCR2/IDD | | P98153 |
| Interleukin 9 receptor | | Q01113 |

Examples of protein and polypeptide drugs and drug targets include hormones that are produced recombinantly, including peptide and protein hormones, such as erythropoietin (EPO) and growth hormone (GH), cytokines, growth factors, such as insulin-like growth factors (IGF-I and IGF-II), KL4, LL-37, hCAP18, surfactant protein C (SP-C) and variants thereof, such as SP-C(Leu), SP-C33, SP-C30 and SP-C33Leu. Other specific examples include SP-C33Leu fused to Mini-B,Mini-BLeu, 1 a AA, 1 b AA, 0 AAAA, 1 a LL, 1 b LL, 0 LLLL or SP-B proteins, optionally via a linker, e.g. Glyₙ, Leuₙ, Gly-Alaₙ or the like. SP-C33Leu may be arranged N-terminal or, preferably, C-terminal to the Mini-B,Mini-BLeu, 1 a AA, 1 b AA, 0 AAAA, 1 a LL, 1b LL, 0 LLLL or SP-B protein. Examples of protein and polypeptide drugs and drug targets according to the invention are listed in Table 6.

**TABLE 6 - Protein and polypeptide drugs and drug targets**

| **Protein** | | **Sequence / Uniprot ID** |
|---|---|---|
| Insulin-like growth factor IA | | P01243 |
| Insulin like growth factor IB | | P05019 |
| Growth hormone 1, variant 1 | | Q61YF1 |
| Growth hormone 1, variant 2 | | Q61YF0 |
| Growth hormone 2, variant 2 | | B1A4H7 |
| Insulin | | P01308 |
| Erythropoietin | | P01588 |
| Coagulation Factor VIII | | P00451 |
| Coagulation Factor IX | | P00740 |
| Prothrombin | | P00734 |
| Serum albumin | | P02768 |
| Antithrombin III | | P01008 |
| Interferon alfa | | P01563 |
| Somatotropin | | P01241 |
| Major pollen allergen Bet v 1-A | | P15494 |
| OspA *(Piscirickettsia salmonis)* | | Q5BMB7 |
| 17 kDa antigen variant of OspA (*P. salmonis)* | | Q9F9K8 |
| Transforming growth factor beta-1 | | P01137 |
| Transforming growth factor beta-2 | | P61812 |
| Transforming growth factor beta-3 | | P10600 |
| Interleukin 1 beta | | P01584 |
| Interleukin 1 alfa | | P01583 |
| Interleukin 2 | | P60568 |
| Interleukin 3 | | P08700 |
| Interleukin 4 | | P05112 |
| Interleukin 5 | | P05113 |
| Interleukin 6 | | P05231 |
| Interleukin 7 | | P13232 |
| Interleukin 8 | | P10145 |
| Interleukin 9 | | P15248 |
| Interleukin 10 | | P22301 |
| Interleukin 12 subunit alfa | | P29459 |
| Interleukin 12 subunit beta | | P29460 |
| Interleukin 18 | | Q14116 |
| Interleukin 21 | | Q9HBE4 |
| Thymic stromal lymphopoietin | | Q969D9 |
| Brichos | | |
| Neuroserpin | | |
| SP-C | FGIPCCPVHLKRLLIVVVVVVLIVVVIVGALLMGL ^{a} | |
| SP-C(Leu) | FGIPSSPVHLKRLKLLLLLLLLILLLILGALLMGL | |
| SP-C33 | IPSSPVHLKRLKLLLLLLLLILLLILGALLMGL | |
| SP-C30 | IPSSPVHLKRLKLLLLLLLLILLLILGALL | |
| SP-C33(Leu) | IPSSPVHLKRLKLLLLLLLLILLLILGALLLGL | |
| LL-37 | LLGDFFRKSKEKIGKEFKRIVQRIKDFLRNLVPRTES | |
| KL4 | KLLLLKLLLLKLLLLKLLLLK | |
| 1aAA | CWLARALIKRIQALIPKGGRLLPQLVARLVLRCS ^{b} | |
| 1bAA | AWLCRALIKRIQALIPKGGRLLPQLVCRLVLRAS ^{c} | |
| 0 AAAA | AWLARALIKRIQALIPKGGRLLPQLVARLVLRAS | |
| 1a LL | CWLLRALIKRIQALIPKGGRLLPQLVLRLVLRCS ^{b} | |
| 1b LL | LWLCRALIKRIQALIPKGGRLLPQLVCRLVLRLS ^{c} | |
| 0 LLLL | LWLLRALIKRIQALIPKGGRLLPQLVLRLVLRLS | |
| | | |
| ^{a} Cys-5 and Cys-6 in native SP-C are palmitoylated ^{b} Cys1-Cys33 linkage ^{c} Cys4-Cys27 linkage | | |

Examples of aggregation-prone proteins and polypeptides include avidin, streptavidin and extracellular, ligand-binding parts of cytokine receptors. Examples of aggregation-prone proteins and polypeptides according to the invention are listed in Table 7.

**TABLE 7 - Aggregation -prone proteins and polypeptides**

| **Protein** | **Uniprot ID / other reference** |
|---|---|
| Streptavidin, *Streptomyces avidinii* | P22629 |
| Streptavidin, *Streptomyces lavendulae* | B8YQ01 |
| Streptavidin V1, *Streptomyces venezuelae* | Q53532 |
| Streptavidin V2, *Streptomyces venezuelae* | Q53533 |
| Putative streptavidin, *Burkholderia mallei* (strain SAVP1) | A1V7Z0 |
| Putative streptavidin, *Burkholderia thailandensis* | Q2T1V4 |
| Putative streptavidin, *Burkholderia mallei* | Q62EP2 |
| Core streptavidin | GenBank: CAA77107.1 |
| M4 (quadruple mutein of streptavidin) | J Biol Chem 280(24): 23225-23231 (2005) |
| Avidin, *Gallus gallus* | P02701 GenBank: CAC34569.1 |
| Actin | P68133 |
| Interleukin 6 receptor subunit alfa | P08887 |
| Interleukin 6 receptor subunit beta | P40189 |
| Interleukin 2 receptor subunit alfa | P01589 |
| Interleukin 2 receptor subunit beta | P14784 |
| Cytokine receptor common subunit gamma | P31785 |
| Green Fluorescent Protein (GFP) | |

Examples of proteases include protease 3C from coxsackie virus or human rhinovirus. Further examples of proteases according to the invention are listed in Table 8.

**TABLE 8 - Proteases**

| **Protease** | **Class** | **Accession no.** |
|---|---|---|
| Trypsin (bovine) | serine | P00760 |
| Chymotrypsin (bovine) | serine | P00766 |
| Elastase (porcine) | serine | P00772 |
| Endoproteinase Arg-C (mouse submaxillary gland) | serine | |
| Endoproteinase Glu-C (V8 protease) *(Staphylococcus aureus)* | serine | P04188 |
| Acylamino-acid-releasing enzyme (porcine) | serine | P19205 |
| Carboxypeptidase *(Penicillium janthinellum)* | serine | P43946 |
| Proteinase K *(Tritirachium album)* | serine | P06873 |
| Subtilisin *(Bacillus subtilis)* | serine | P04189 P29122 |
| Carboxypeptidase Y (yeast) | serine | P00729 |
| Endoproteinase Lys-C *(Lysobacter enzymogenes)* | serine | S77957 |
| Enteropeptidase (human) | serine | P98073 |
| Prothrombin | serine | P00734 |
| Factor X | serine | P00742 |
| Pepsin | aspartic | P00791 P00790 |
| Cathepsin D (human) | aspartic | P07339 |
| HIV-1 protease | aspartic | Q9YQ34 |
| Cathepsin C | cysteine | |
| Clostripain (endoproteinase-Arg-C) *(Clostridium histolyticum)* | cysteine | P09870 |
| Papain *(Carica papaya)* | cysteine | P00784 |
| Protease 3C | cysteine | Q04107 |
| Tobacco Etch virus (TEV) | cysteine | Q0GDU8 |
| Thermolysin (Bacillus thermo-proteolyticus) | metallo | P00800 |
| Endoproteinase Asp-N (Pseudomonas fragi) | metallo | Q9R4J4 |
| Carboxypeptidase A (bovine) | metallo | P00730 |
| Carboxypeptidase B (porcine) | metallo | P00732 |
| IgA protease | metallo | Q97QP7 |

In preferred embodiments of the invention, the desired protein or polypeptide is selected from surfactant protein B (SP-B) and variants thereof, such as Mini-B, Mini-B27, Mini-BLeu, KL4, LL-37, hCAP18, and surfactant protein C (SP-C) and variants thereof, such as SP-C(Leu), SP-C33, SP-C30 and SP-C33Leu. Other preferred non-spidroin proteins according to the invention are neuroserpin, GFP, and the 1 a AA, 1 b AA, 0 AAAA, 1 a LL, 1 b LL and 0 LLLL proteins.

In certain preferred embodiments of the invention, the fusion protein is selected from the group consisting of SEQ ID NOS 6, 8, 10, and 12-34; and proteins having at least 80%, preferably at least 90%, more preferably at least 95% identity, to any of these proteins.

According to one aspect, the present invention provides a composition comprising an aqueous solution of a protein according to the invention. In a preferred embodiment, the composition is consisting of an aqueous solution of a protein according to the invention. It is preferred that the protein is a fusion protein according to the invention. It is preferred that the pH of the composition is 6.3 or lower, such as 4.2-6.3.

According to another aspect, the present invention provides an isolated nucleic acid encoding a protein according to the invention. In a preferred embodiment, the isolated nucleic acid is selected from the group consisting of SEQ ID NOS 2, 5, 7, 9 and 11.

According to one aspect, the present invention provides a novel use of at least one moiety of 100-160 amino acid residues having at least 80% identity with SEQ ID NO 1, wherein the amino acid residue corresponding to position 72 in SEQ ID NO 1 is not Ala or Gly, as a moiety in a fusion protein for enhancing the solubility of another moiety in the fusion protein, which is a desired protein or polypeptide. Preferred features of the inventive moiety are presented hereinabove.

In one preferred embodiment, the solubility-enhancing moiety is used for production of the desired protein or polypeptide. In another preferred embodiment, the solubility-enhancing moiety is used for studying or characterizing the desired protein or polypeptide.

An advantageous use of the inventive moiety is as a solubility-enhancing moiety in a fusion protein which is subjected to a pH of 6.3 or lower, such as 4.2-6.3. This specific variant of the N-terminal (NT) fragment of a spider silk protein is present as a soluble monomer regardless of the pH of the surrounding aqueous medium. Wildtype NT forms dimers at a pH interval of 4.2-6.3 which increases the risk of undesirable aggregation of the fusion proteins. This is a useful pH interval for the functionality and stability of certain desirable proteins and polypeptide. It is also a useful pH interval for certain purification protocols, e.g. when using cation exchange as a purification principle.

According to another aspect, the present invention provides a method of producing a fusion protein. The first step involves expressing in a suitable host a fusion protein according to the invention. Suitable hosts are well known to a person skilled in the art and include e.g. bacteria and eukaryotic cells, such as yeast, insect cell lines and mammalian cell lines. Typically, this step involves expression of a nucleic acid molecule which encodes the fusion protein in *E. coli.*

The second method step involves obtaining a mixture containing the fusion protein. The mixture may for instance be obtained by lysing or mechanically disrupting the host cells. The mixture may also be obtained by collecting the cell culture medium, if the fusion protein is secreted by the host cell. The thus obtained protein can be isolated using standard procedures. If desired, this mixture can be subjected to centrifugation, and the appropriate fraction (precipitate or supernatant) be collected. The mixture containing the fusion protein can also be subjected to gel filtration, chromatography, e.g. cation exchange chromatography, dialysis, phase separation or filtration to cause separation. Optionally, lipopolysaccharides and other pyrogens are actively removed at this stage. If desired, linker peptides may be removed by cleavage in this step. In a preferred embodiment, the obtained mixture comprises the fusion protein dissolved in a liquid medium, typically a salt buffer or cell culture medium. In one preferred embodiment, the mixture has a pH of 6.3 or lower, such as 4.2-6.3.

According to a related aspect, the present invention provides a method of producing a desired protein or polypeptide. The first step involves expressing in a suitable host a fusion protein according to the invention. Suitable hosts are well known to a person skilled in the art and include e.g. bacteria and eukaryotic cells, such as yeast, insect cell lines and mammalian cell lines. Typically, this step involves expression of a nucleic acid molecule which encodes the fusion protein in *E. coli.*

The second method step involves obtaining a mixture containing the fusion protein. The mixture may for instance be obtained by lysing or mechanically disrupting, e.g. sonicating, the host cells. The mixture may also be obtained by collecting the cell culture medium, if the fusion protein is secreted by the host cell. The thus obtained protein can be isolated using standard procedures. If desired, this mixture can be subjected to centrifugation, and the appropriate fraction (precipitate or supernatant) be collected. The mixture containing the fusion protein can also be subjected to gel filtration, chromatography, e.g. cation exchange chromatography, dialysis, phase separation or filtration to cause separation. Optionally, lipopolysaccharides and other pyrogens are actively removed at this stage. If desired, linker peptides may be removed by cleavage in this step. As set out above, this may be the most suitable form of the desired protein or polypeptide, i.e. as part of a fusion protein. It may provide a suitable handle for purification and detection and/or provide desirable properties, e.g. stability and in particular solubility.

In a preferred embodiment, the method may also comprise the step of cleaving the fusion protein to provide the desired protein or polypeptide. In this embodiment, the fusion protein is comprising at least one cleavage site arranged between at least one desired protein or polypeptide moiety and at least one solubility-enhancing moiety. In a typical fusion protein, this implies the presence of a single cleavage site between the solubility-enhancing moiety or moieties and the desired protein or polypeptide. Cleavage may be achieved using standard procedures, for instance cleavage by cyanogen bromide (CNBr) after Met residues, cleavage by hydroxylamine between Asn and Gly residues, cleavage by protease 3C between Gln and Gly residues at -XLETLFQGX- sites, and at various other protease sites that are well known to the person skilled in the art.

The thus obtained desired protein or polypeptide can be isolated using standard procedures. If desired, this mixture can be subjected to centrifugation, and the appropriate fraction (precipitate or supernatant) be collected. The mixture containing the desired protein or polypeptide can also be subjected to gel filtration, chromatography, dialysis, phase separation or filtration to cause separation. Optionally, lipopolysaccharides and other pyrogens are actively removed at this stage. If desired, linker peptides may be removed by cleavage in this step.

In a preferred embodiment, the obtained mixture comprises the fusion protein dissolved in a liquid medium, typically a salt buffer or cell culture medium. In one preferred embodiment, the mixture has a pH of 6.3 or lower, such as 4.2-6.3. This specific variant of the N-terminal (NT) fragment of a spider silk protein is present as a soluble monomer regardless of the pH of the surrounding aqueous medium. Wildtype NT forms dimers at a pH interval of 4.2-6.3 which increases the risk of undesirable aggregation of the fusion proteins. This is a useful pH interval for the functionality and stability of certain desirable proteins and polypeptide, e.g. amyloid-forming or aggregation-prone proteins/polypeptides. It is also a useful pH interval for certain purification protocols, e.g. when using cation exchange as a purification principle. In a preferred embodiment, step b) further involves purification of the fusion protein on a cation exchange medium.

Thus, the fusion protein is typically obtained as a solution in a liquid medium. By the terms "soluble" and "in solution" is meant that the fusion protein is not visibly aggregated and does not precipitate from the solvent at 60 000×g. The liquid medium can be any suitable medium, such as an aqueous medium, preferably a physiological medium, typically a buffered aqueous medium, such as a 10-50 mM Tris-HCl buffer or phosphate buffer.

It has been advantageously been found that the presence of the solubility-enhancing moiety according to the invention improves the stability of the desired protein/polypeptide and prevents moiety dimer formation under these conditions. This can be advantageous when immediate polymerisation may be undesirable, e.g. during protein purification or in preparation of large batches. In particular, this is advantageous for methods according to the invention which are comprising at least one step involves subjecting the fusion protein to a pH of 6.3 or lower, such as 4.2-6.3. As set out above, this specific variant of the N-terminal (NT) fragment of a spider silk protein is present as a soluble monomer regardless of the pH of the surrounding aqueous medium. Wildtype NT forms dimers at a pH interval of 4.2-6.3 which increases the risk of undesirable aggregation of the fusion proteins. This is a useful pH interval for the functionality and stability of certain desirable proteins and polypeptide, e.g. amyloid-forming or aggregation-prone proteins/polypeptides.

The present invention will in the following be further illustrated by the following non-limiting examples.

### Examples

### Example 1 - Production of NT^{A72R} and NT

An expression vector was constructed to produce NT as a C-terminal fusion to His₆-thioredoxin-His₆. The mutation A72R (SEQ ID NO 5) was introduced in the NT gene, and the mutant (SEQ ID NO 6) and wt NT were expressed and purified. *E. coli* BL21 (DE3) cells were grown at 37°C in the presence of kanamycin, either in minimal medium M9 containing ¹⁵N-ammonium acetate and ¹³C-glucose (for NMR studies), or in LB medium (for all other analyses). Protein expression was induced by addition of isopropyl-β-D-thiogalactopyranoside (IPTG), and the cells were thereafter kept for 4 h at 30°C. Cells were harvested and resuspended in 20 mM TRIS buffer, pH 8. After addition of lysozyme and DNase I, the suspension was centrifuged at 20 000×g, and the supernatant was loaded on a Ni-NTA sepharose column (GE Healthcare). After washing, bound proteins were eluted with 300 mM imidazole, and the fractions containing target protein were pooled and dialyzed against 20 mM TRIS, pH 8, containing 1:1000 (w/w) thrombin to release the mutant (SEQ ID NO 1) and wt (SEQ ID NO 3) products. Finally, the cleavage mixture was loaded on to a second Ni-NTA sepharose column and the flow-through was collected. Both products are highly soluble and produced in high yields, above 50 mg/L culture of purified target protein, and at least 150 mg/L culture of purified protein.

### Example 2 - Structural comparison of NT and NT^{A72R}

The proteins NT (SEQ ID NO 3) and NT^{A72R} (SEQ ID NO 1) were subjected to comparative structural studies. The data confirm that lowering the pH from 7 to 6 entails a switch from monomer to stable dimer of wt NT, and shows that NT^{A72R} is unable to form stable dimers in the same manner as wildtype NT.

### A) Fluorescence emission

Fluorescence emission spectra between 300 and 400 nm were recorded for the proteins NT and NT^{A72R} at 5 µM concentration in 10 mM Hepes/Mes, pH values between 5.6 and 7.6, using a Fluorolog-3 instrument, excitation wavelength 280 nm, emission and excitation slit widths 5 nm.

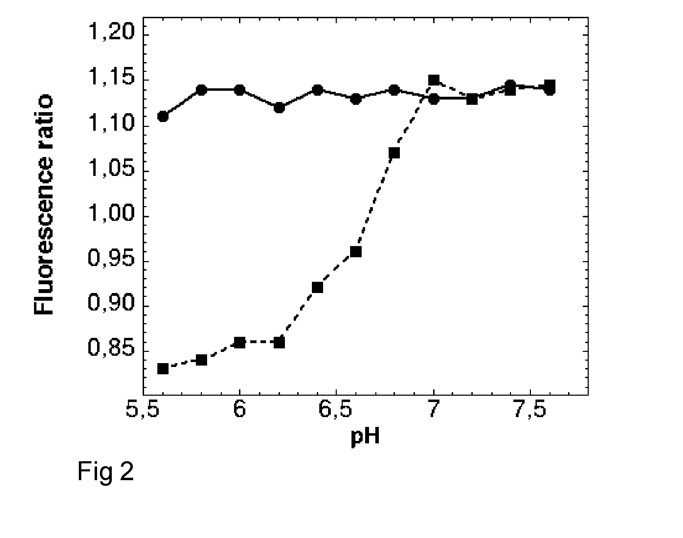

The ratio of fluorescence emission at 338 nm and 353 nm vs pH for NT^{A72R} (solid line) and wild type NT (dotted line) are shown in Fig. 2. The tryptophan fluorescence spectroscopy of NT^{A72R} shows no significant changes between pH 7 and pH 6, while wild type NT shows a 15 nm red-shift in fluorescence emission.

### B) Hydrogen-Deuterium Exchange-Mass Spectrometry (HDX-MS)

For HDX-MS, NT^{A72R}, 1 mg/mL, was incubated at 22°C in 90% D₂O in 18 mM Tris-HCl at pH 6.0 or 7.0. Incubations were started by diluting a 10 mg/mL protein solution 1:10 in buffer prepared in 99.9% D₂O (Cambridge Isotopes, Andover, MA). Aliquots were collected from 1 min and onwards, quenched by addition of trifluoroacetic acid (TFA) (Merck, Darmstadt, Germany), and frozen in liquid nitrogen. Samples were kept in liquid nitrogen until analyzed. For MS, aliquots of deuterated NT^{A72R} were thawed and injected into an HPLC system submersed in an ice bath. Protein samples were digested online in a Porozyme Immobilized Pepsin Cartridge (Applied Biosystems, Foster City, CA) operated at 17 µL/min in 0.05% TFA. Peptic peptides were desalted using a Waters Symmetry C₁₈ trap column and eluted in a single step with 70% acetonitrile containing 0.1 % formic acid at a flow rate of 17 µL/min.

Samples were delivered to the mass spectrometer through a tapered tip emitter with an opening of 50 µm (New Objective, Milford, MA) coupled to the HPLC via a T-connector. Spectra were acquired in the positive-ion mode on a Waters Ultima API mass spectrometer (Waters, Milford, MA) equipped with a Z-spray source. The source temperature was 80°C, the capillary voltage 1.7 kV and the cone and RF lens 1 potentials were 100 and 38 V, respectively. The mass spectrometer was operated in single-reflector mode to achieve a resolution of 10 000 (full width half maximum definition). The mass scale was calibrated using [Glu1]-fibrinopeptide B. Scans were acquired for 5 min at a rate of one scan per 2 sec between 200 and 2000 m/z. To correct for back exchange, fully deuterated protein was prepared by incubating a sample of NT^{A72R} for 4 h at 50°C. Peptic peptides were identified based on a map of pepsin-digested undeuterated material using automated LC-MS/MS analysis with a Waters NanoAcquity system (Waters, Milford, MA). Peptide sequences were identified by individual analysis of collision induced dissociation (CID) spectra using the Waters MassLynx and ProteinLynx software packages. Deuteration rates were determined by calculating the average m/z value of the isotope envelope centroids using the Waters MassLynx software package.

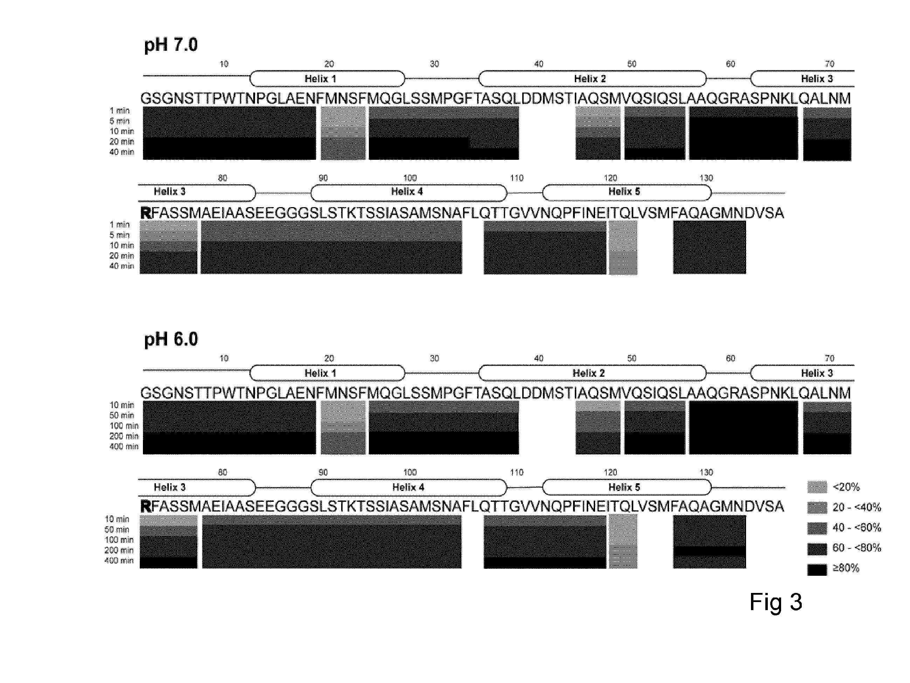

HDX-MS shows no difference in NT^{A72R} between pH 7.0 and 6.0 (Fig. 3). Deuterium incorporation for each peptic peptide was observed. HDX-MS of NT^{A72R} did not show any significant changes between pH 7 and pH 6, while wildtype NT shows a reduced HDX, in particular in helix 3 spanning amino acid residues 63-84. The reduced HDX of wildtype NT indicates global stabilisation at low pH and is in line with the increased melting temperature of NT at low pH.

### C) Nuclear Magnetic Resonance (NMR)

NMR experiments were performed at 298 K on a Varian Unity Inova 600 MHz spectrometer equipped with an HCN triple resonance PFG cold probe. The NMR sample of wt NT sample contained 1.9 mM protein, 20 mM sodium phosphate (pH 7.2), 300 mM NaCl, 5% (v/v) D₂O and 0.03% NaN₃ (w/v).

The ¹⁵N-HSQC spectrum of NT^{A72R} showed narrow and well-dispersed resonances even in the presence of low salt concentrations at pH 7.0 (data not shown), indicating that the protein is folded and monomeric at these conditions. Nearly complete resonance assignments for the polypeptide backbone nuclei (HN, ¹⁵N, ¹³C^{α}, ¹³C' and ¹³C^{β}) were obtained through analysis of triple-resonance 3D NMR spectra. Amino acid side chains were assigned using 3D ¹⁵N-NOESY-HSQC and two ¹³C-NOESY-HSQC spectra. The following two- and three-dimensional (2D and 3D) spectra were recorded, with the number of complex points and mixing times as given in parenthesis: ¹⁵N-HSQC (1024 x 256), HNCA (1024 x 32 x 64), CBCA(CO)NH (1024 x 32 x 64), ¹⁵N-resolved NOESY-HSQC (1024 x 32 x 160, 80 ms), ¹³C(aliphatic)-resolved NOESY-HSQC (768 x 60 x 150, 80 ms) and ¹³C(aromatic)-resolved NOESY-HSQC (1024 x 48 x 128, 80 ms). Additionally, a ¹⁵N{1H} heteronuclear NOE experiment was recorded for characterization of protein backbone dynamics in the pico-to-nanosecond timescale using the TROSY-based pulse sequence, a saturation period of 3.0 s and a total interscan delay of 5.0 s. All data were processed with NMRPipe and analyzed using CARA.

Analysis of ¹³C^{α,β} chemical shift deviations from random coil values indicated the presence of five helices at locations almost identical to those in the wt NT dimer (data not shown). Structure calculation then resulted in a high quality structure as shown by the structural statistics for the final 20 conformers representing the NMR structure of NT^{A72R}.

To confirm that the structure of NT^{A72R} is representative of the monomeric form of wt NT, we determined the wt NT solution structure at pH 7.2 in the presence of 300 mM NaCl. These conditions enabled the recording of high-quality NMR data for wt NT (data not shown). The same procedure as for NT^{A72R} was used to obtain resonance assignments and to determine the structure. Although a higher number of NOE distance constraints could be obtained than for NT^{A72R}, the resulting wt NT monomer structure is of equal quality to the NT^{A72R} structure. Furthermore, it is essentially identical with the NMR and X-ray structures of NT^{A72R}, including a highly similar side chain arrangement near Trp10. This leads us to conclude that the NT^{A72R} mutation successfully locks the protein in the wildtype-like monomeric form.

An overlay of 600 MHz ¹⁵N-HSQC NMR spectra of NT^{A72R} at pH 7.0 and pH 6.0 shows no significant changes in the chemical shifts of backbone and side chain amide resonances (data not shown). ¹⁵N-HSQC NMR spectroscopy of NT^{A72R} did not show any significant changes between pH 7 and pH 6, while wildtype NT shows chemical shift changes for residues at the dimerization site between pH 7 and 6 (Hagn, F et al. Angew. Chem. Int. Ed. Engl. 50, 310-313 (2011)).

### D) Crystallization

Crystals of NT^{A72R} were grown using the hanging drop vapor diffusion method at 293 K. Single crystals belonging to space group P2₁ (a = 42.65 A, b = 33.24 Å, c = 44.75 Å, β = 115.4°) were obtained in 0.1 M Bis Tris propane pH 6.5, 0.25 M NaBr, 20% PEG 3350. There is one molecule in the asymmetric unit.

Initial attempts to solve the crystal structure of NT^{A72R} by molecular replacement using a monomer from the wt dimer structure (pdb id 3LR2) were not succesful, suggesting that the monomer may have a conformation distinct from that observed in the dimer. The structure of NT^{A72R} was solved by molecular replacement using AMoRe with the A (central) chain from the NMR solution structure as search model. Repetitive manual rebuilding in o³⁵ interspersed with refinement using Refmac 5³⁶ resulted in a model comprising residues 7-85 and 88-130 and water molecules, with final R = 0.239 and R_{free} = 0.174 and good stereochemistry (data not shown). Model validation was carried out using MolProbity. All residues have main chain torsion angles in the allowed region of the Ramachandran plot (99.15% of residues in the favoured region).

Thus, the crystal structure of NT^{A72R} was solved to 1.7 Å resolution by molecular replacement using the NMR structure as the search model. As expected, the mutated NT is monomeric, with one monomer in the asymmetric unit. The first six and the last seven residues of the polypeptide chain as well as two glycine residues in the loop between helices 3 and 4 are disordered and have not been modeled. These residues also show increased mobility and disorder in solution as manifested by ¹⁵N{1H}-NOE values below 0.6 and higher local RMSD values. The characteristic up-and-down five-helix bundle (residues 13-130) of wt NT dimer is apparent in both NT^{A72R} structures, which agree well with each other and yield a backbone all-heavy-atom RMSD of 0.95 Å, when superimposed.

### Example 3 - Comparison of monomeric and dimeric NT conformations.

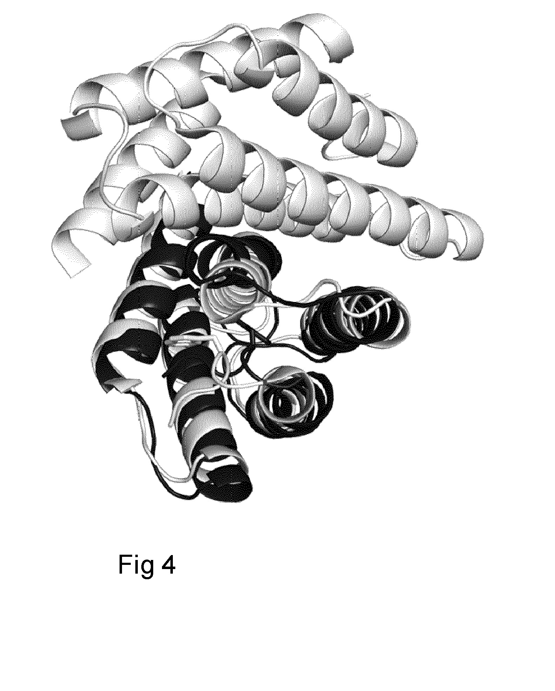

Superpositioning in Fig. 4 of the monomeric (black) and dimeric (white/grey) NT subunit structures reveals that the helices of the five-helix bundle are significantly shifted in the two forms (Fig 4). Helix 2 (residues 35-60) and 3 (residues 63-84) form one unit, while helix 1 (residues 10-30), 4 (residues 90-110) and 5 (residues 113-130) form a second, with the two units shifted approximately as rigid bodies with respect to each other. In agreement with this, superpositioning of pairs of helices in the wt dimer subunit and the wt monomer reveal significantly larger Cα position RMSD values for helix pairs 1 and 3, 2 and 5, 3 and 4, and 3 and 5. The helix dislocations are caused by the burial of Trp10 in the protein core of monomeric NT and NT^{A72R}, where it interacts with mostly hydrophobic residues as opposed to its more hydrophilic environment in dimeric NT. This explains the tryptophan fluorescence red-shift observed upon lowering the pH of wt NT samples (i.e. upon dimerization) and validates its use as a probe for monitoring NT assembly.

The subunit interface in dimeric NT is formed by helices 2, 3, and 5. Since helix 2 and 3 form a more or less rigid unit, two wt subunits in the monomeric conformation might potentially be able to dimerize via these two helices. However, modeling shows that this would require significant movement of helix 5 to resolve clashes.

In dimeric NT, the two highly conserved residues D40 at the beginning of helix 2 and E84 at the end of helix 3 form an intramolecular handshake interaction. This interaction is lost in monomeric NT and NT^{A72R}, where the two carboxylates are more than 10 Å apart in monomeric NT and NT^{A72R}. This is accompanied by unwinding of the last half-turn of helix 3 in monomeric NT. Previous results indicate that NT can interconvert between different conformations and quaternary states. The x-ray structure of wt NT shows a dimer in which a Asp40-Glu84 handshake interaction, intersubunit charge interactions, as well as the C-terminal region show conformational polymorphisms.

The present study clearly shows that NT can exist as a monomer, be locked in a monomer form, and defines the conformational changes required for conversion to the dimer observed by x-ray crystallography. The folds of monomeric and dimeric NT domains are very similar and the locations of the α-helices in the polypeptide chains are virtually identical, but distinct changes in local conformations are seen. Relocation of the single tryptophan of NT, Trp10, from a buried to an exposed site is a main difference and is readily monitored by fluorescence spectroscopy (Fig. 2). Trp10 is present in most of the sequenced species variants of NT, but can be replaced with Phe in some species.

### Example 4 - Production of a desired protein from a NT^{A72R} fusion protein

Expression vectors were constructed to produce SP-C33Leu, miniBLeu and proSP-C(86-197), respectively, as C-terminal fusions to HisNT^{A72R} (SEQ ID NOS 7, 9 and 11, respectively). The corresponding fusion proteins HisNT^{A72R}MetSP-C33Leu (SEQ ID NO 8), HisNT^{A72R}MetMiniBLeu (SEQ ID NO 10) and HisNT^{A72R}proSP-C(86-197) (SEQ ID NO 12) were expressed and purified essentially as set out in Example 1. The yields obtained were:

| | |
|---|---|
| HisNT^{A72R}MetSP-C33Leu | above 300 mg/L culture of purified fusion protein, and above 50 mg/L of purified target protein |
| HisNT^{A72R}MetMiniBLeu | above 50 mg/L culture of fusion protein |
| HisNT^{A72R}proSP-C(86-197) | above 0.5 mg/L culture of fusion protein |

### Example 5 - Production of a desired protein from a NT^{A72R} fusion protein

Expression vectors are constructed to produce a number of desirable proteins and polypeptides as a C-terminal fusion to His-protease 3C cleavage site-NT^{A72R}- TEV protease cleavage site. The corresponding fusion proteins (SEQ ID NO 13-34 and corresponding sequences for fusion proteins incorporating the desirable proteins/polypeptide 4repCT, LL37/hCAP18, SPC-33, Bri2(76-231), Bri2-NL(90-236), Bri2(113-266), Bri2(125-231), Bri2(232-236), and Bri2-hairpin(244-266)) are expressed, and the desirable proteins and polypeptides are released and purified essentially as set out in Example 1.

**Desirable proteins:**

| | |
|---|---|
| Construct ID | SGC Protein Family |
| ACOT9A-c009 | Lipid signalling-Other |
| ALKA-c023 | Kinase-TK |
| APOBEC3AA-c002 | Cytosine deaminases |
| BIRC1A-c050 | Apoptosis-Inflammation Domains |
| CSADA-c006 | aa metabolic enzymes |
| DDX53A-c006 | ATPases - DExD-DExH RNA helicases |
| DDX53A-c013 | ATPases - DExD-DExH RNA helicases |
| DOCK1A-c021 | Lipid signalling-Other |
| GARTA-c015 | Nucleotide metabolism-Others |
| GARTA-c017 | Nucleotide metabolism-Others |
| GLE1A-c007 | Miscellaneous |
| GMPSA-c020 | Nucleotide metabolism-Others |
| ITM2BA-c006 | BRICHOS |
| ITPKCA-c208 | Kinase-PIK |
| MYO10A-c013 | Lipid signalling-Other |
| NUDT6A-c008 | Nucleotide metabolism-NUDIX |
| NUDT6A-c032 | Nucleotide metabolism-NUDIX |
| PIP5K2CA-c002 | Kinase-PIK |
| ROR2A-c013 | Kinase-TK |
| SKILA-c017 | Receptor Signaling Domains |
| UMPSA-c001 | Nucleotide metabolism-Others |

## Claims

1. A protein comprising a moiety of 100-160 amino acid residues having at least 80% identity with SEQ ID NO 1, wherein the amino acid residue corresponding to position 72 in SEQ ID NO 1 is not Ala or Gly.

2. A protein according to claim 1, wherein the amino acid residue corresponding to position 72 in SEQ ID NO 1 is selected from the group consisting of Arg, Lys, His, Glu, Asp, Gln, Asn, Tyr, Thr and Ser.

3. A protein according to claim 2, wherein the amino acid residue corresponding to position 72 in SEQ ID NO 1 is selected from the group consisting of Arg, Lys, His, Glu and Asp.

4. A protein according to claim 3, wherein the amino acid residue corresponding to position 72 in SEQ ID NO 1 is Arg.

5. A protein according to any preceding claim, which is a fusion protein comprising
(i) at least one moiety according to any preceding claim which is a solubility-enhancing moiety; and
(ii) at least one moiety which is a desired protein or polypeptide.

6. A fusion protein according to claim 5, further comprising
(iii) at least one cleavage site arranged between at least one desired protein or polypeptide moiety and at least one solubility-enhancing moiety.

7. A fusion protein according to any one of claims 5-6, wherein the desired protein or polypeptide is selected from the group consisting of amyloid-forming proteins and polypeptides, disulphide-containing proteins and polypeptides, apolipoproteins, membrane proteins and polypeptides, protein and polypeptide drugs and drug targets, aggregation-prone proteins and polypeptides, and proteases.

8. A fusion protein according to claim 7, wherein the desired protein or polypeptide is selected from the group consisting of Aβ-peptide, IAPP, PrP, α-synuclein, calcitonin, prolactin, cystatin, ATF and actin; SP-B, mini-BLeu, α-defensins and β-defensins; class A-H apolipoproteins; LL-37, hCAP18, SP-C, SP-C33, SP-C33Leu, Brichos, GFP, neuroserpin; hormones, including EPO and GH, and growth factors, including IGF-I and IGF-II; avidin and streptavidin; and protease 3C.

9. A composition comprising an aqueous solution of a protein according to any one of claims 1-8, wherein the pH of said composition is 6.3 or lower, such as 4.2-6.3.

10. An isolated nucleic acid encoding a protein according to any one of claims 1-8.

11. Use of at least one moiety according to any one of claims 1-4 as a moiety in a fusion protein for enhancing the solubility of another moiety in the fusion protein, which is a desired protein or polypeptide.

12. Use according to claim 11, wherein said use involves subjecting the fusion protein to a pH of 6.3 or lower, such as 4.2-6.3.

13. A method of producing a fusion protein, comprising the following steps:
a) expressing in a suitable host a fusion protein according to any one of claims 5-8; and
b) obtaining a mixture containing the fusion protein, and optionally isolating the fusion protein.

14. A method of producing a desired protein or polypeptide, comprising the following steps:
a) expressing in a suitable host a fusion protein according to any one of claims 5-8;
b) obtaining a mixture containing the fusion protein or polypeptide, and optionally isolating the fusion protein or polypeptide; and
c) cleaving the fusion protein to provide the desired protein or polypeptide; and optionally
d) isolating the desired protein or polypeptide.

15. A method according to any one of claims 13-14, wherein at least one step involves subjecting the fusion protein to a pH of 6.3 or lower, such as 4.2-6.3.
